# EUROPEAN PATENT SPECIFICATION

(11) **EP 3 207 889 B1**
(45) Date of publication and mention of the grant of the patent: **31.07.2019**
(21) Application number: 16779987.3
(22) Date of filing: 08.04.2016
(51) Int. Cl.: A61B 18/14, A61B 18/08, A61B 17/28

(54) **GRASPING TREATMENT UNIT**
GREIFBEHANDLUNGSEINHEIT
UNITÉ DE TRAITEMENT DE PRÉHENSION

(30) Priority: 13.04.2015 JP 2015081969
(43) Date of publication of application: 23.08.2017
(73) Proprietor: Olympus Corporation, Hachioji-shi, Tokyo 192-8507 (JP)
(72) Inventor: TAKEI, Yusuke, Hachioji-shi, Tokyo 192-8507 (JP); TANAKA, Kazuhiro, Hachioji-shi, Tokyo 192-8507 (JP); TAKASHINO, Tomoyuki, Hachioji-shi, Tokyo 192-8507 (JP)
(74) Representative: Gunzelmann, Rainer
(86) International application number: PCT/JP2016/061588
(87) International publication number: WO 2016/167196

(56) References cited:
- JP-A- 2008 504 084
- JP-A- 2009 090 060
- JP-A- 2012 055 691
- US-A- 5 908 420
- US-A1- 2001 037 109
- US-A1- 2003 171 747

## Description

### Technical Field

The present invention relates to a grasping treatment unit capable of grasping a treated target between a blade (first grasping piece) and a jaw (second grasping piece).

### Background Art

Jpn. Pat. Appln. KOKAI Publication No. 2001-198137 discloses a grasping treatment unit capable of grasping a treated target between a first grasping portion (first grasping piece) and a second grasping portion (second grasping piece). The first grasping portion is provided with a heating body, and a heat transmitting plate (blade) to which heat is transmitted from the heating body. In the heat transmitting plate, a top sharply formed toward the second grasping portion extends from a proximal portion to a distal portion. The first grasping portion (blade) and the second grasping portion (jaw) is closed with respect to each other, and the treated target is then grasped in a state where heat is released from the heat transmitting plate toward the second grasping portion, whereby a portion in the treated target which contacts the top receives high pressure and is thus cut off. On both sides of the top in a width direction, pressure from the heat transmitting plate to the treated target is lower than at the top, and the treated target is therefore not cut off and is sealed.

### Summary of Invention

There is a case where a treated target longer than the entire length of the top is cut by use of the grasping treatment unit according to Jpn. Pat. Appln. KOKAI Publication No. 2001-198137. In this case, it is possible that a seal allowance (seal portion) may not be formed between a portion with which the distal end of the top comes into contact in a longitudinal direction of the heat transmitting plate and an uncut portion of the treated target which is not cut in the vicinity of the top. Further related prior art can be found in patent publication US 5,908,420 A and patent applications US 2001/037109 A1, US 2003/171747 A1.

The present invention has been made in view of the foregoing problems, and its object is to provide a grasping treatment unit wherein a region between a position in a treated target which comes into contact with a distal end of a top of a blade and an uncut portion in its vicinity is suitably sealed.

To solve the above mentioned problems, according to one aspect of the invention, a grasping treatment unit including the features of claim 1 is provided. Further aspects of the invention are defined in the dependent claims.

### Brief Description of Drawings

FIG. 1 is a perspective view schematically showing a grasping treatment system according to a first embodiment;
FIG. 2 is a sectional view schematically showing, through a section perpendicular to a width direction, the configuration of a distal portion of a grasping treatment instrument including a grasping treatment unit according to the first embodiment;
FIG. 3 is a sectional view schematically showing, through a section perpendicular to a longitudinal direction, a proximal portion of the grasping treatment unit according to the first embodiment;
FIG. 4 is a sectional view schematically showing, through the section perpendicular to the longitudinal direction, a distal portion of the grasping treatment unit according to the first embodiment;
FIG. 5 is a perspective view schematically showing the configuration of a pad portion of a second grasping piece according to the first embodiment;
FIG. 6 is a schematic view showing a treatment which is conducted by grasping, as a treated target, a blood vessel having a diameter larger than the entire length of the second grasping piece in the longitudinal direction according to the first embodiment;
FIG. 7 is a schematic view showing a treatment which grasps a thin membranous tissue between a first grasping piece and the second grasping piece according to the first embodiment;
FIG. 8 is a sectional view schematically showing the first grasping piece through the section perpendicular to the longitudinal direction according to a first modification of the first embodiment;
FIG. 9 is a sectional view schematically showing, through the section perpendicular to the longitudinal direction, the proximal portion of the grasping treatment unit according to a second modification of the first embodiment;
FIG. 10 is a sectional view schematically showing, through the section perpendicular to the longitudinal direction, the distal portion of the grasping treatment unit according to the second modification of the first embodiment;
FIG. 11 is a sectional view schematically showing, through the section perpendicular to the longitudinal direction, the proximal portion of the grasping treatment unit according to a third modification of the first embodiment;
FIG. 12 is a sectional view schematically showing, through the section perpendicular to the longitudinal direction, the proximal portion of the grasping treatment unit according to the third modification of the first embodiment;
FIG. 13 is a sectional view schematically showing, through the section perpendicular to the width direction, the configuration of the pad portion of the second grasping piece according to a fourth modification of the first embodiment;
FIG. 14 is a sectional view schematically showing, through the section perpendicular to the width direction, the configuration of the pad portion of the second grasping piece according to a fifth modification of the first embodiment;
FIG. 15 is a sectional view schematically showing, through the section perpendicular to the width direction, the configuration of the pad portion of the second grasping piece according to a sixth modification of the first embodiment;
FIG. 16 is a perspective view schematically showing the configuration of the first grasping piece according to a seventh modification of the first embodiment;
FIG. 17 is a perspective view schematically showing the configuration of the second grasping piece according to an eighth modification of the first embodiment;
FIG. 18 is a sectional view schematically showing, through the section perpendicular to the width direction, the configuration of the distal portion of the grasping treatment instrument including the grasping treatment unit according to a second embodiment;
FIG. 19 is a sectional view schematically showing, through the section perpendicular to the longitudinal direction, the proximal portion of the grasping treatment unit according to the second embodiment;
FIG. 20 is a sectional view schematically showing, through the section perpendicular to the longitudinal direction, the distal portion of the grasping treatment unit according to the second embodiment;
FIG. 21 is a perspective view schematically showing the configurations of the pad portion and a jaw electrode portion of the second grasping piece according to the second embodiment;
FIG. 22 is a sectional view schematically showing, through the section perpendicular to the longitudinal direction, the distal portion of the grasping treatment unit according to the first modification of the second embodiment;
FIG. 23 is a perspective view schematically showing the configurations of the pad portion and the jaw electrode portion of the second grasping piece according to the first modification of the second embodiment;
FIG. 24 is a sectional view schematically showing the first grasping piece through the section perpendicular to the longitudinal direction according to a certain modification of the first embodiment and the second embodiment;
FIG. 25 is a sectional view schematically showing the first grasping piece through the section perpendicular to the longitudinal direction according to another certain modification of the first embodiment and the second embodiment different from that in FIG. 24; and
FIG. 26 is a sectional view schematically showing the first grasping piece through the section perpendicular to the longitudinal direction according to a certain modification of the first embodiment and the second embodiment different from those in FIG. 24 and FIG. 25.

### Description of Embodiments

### (First Embodiment)

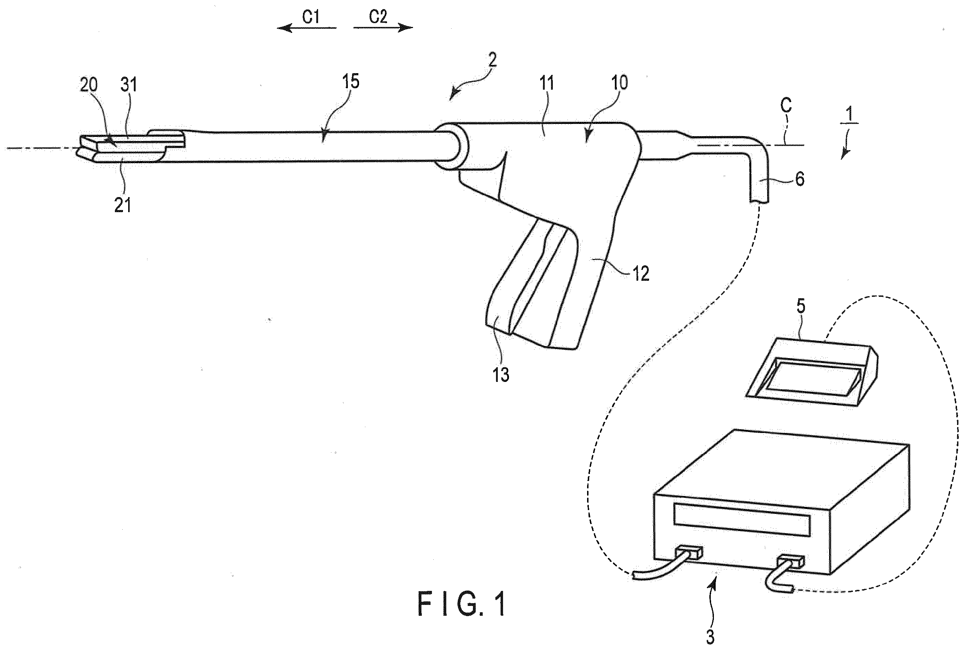

A first embodiment of the present invention is described with reference to FIG. 1 to FIG. 7.

FIG. 1 is a view showing a grasping treatment system (treatment system) 1. As shown in FIG. 1, the grasping treatment system 1 includes a grasping treatment instrument (energy treatment instrument) 2, an energy source unit 3, and an energy operation input component 5 electrically connected to the energy source unit 3. The grasping treatment instrument 2 and the energy source unit 3 are connected to each other via a cable 6. The grasping treatment instrument 2 has a longitudinal axis C. When a direction parallel to the longitudinal axis C is a longitudinal direction, one side of the longitudinal direction is a distal side (an arrow C1 side in FIG. 1), and the side (an arrow C2 side in FIG. 1) opposite to the distal side is a proximal side. The energy source unit 3 is, for example, an energy controller, includes an electric power supply and a conversion circuit which converts electric power from the electric power supply into heat generating electric power, includes a processor or an integrated circuit including a central processing unit (CPU) or an application specific integrated circuit (ASIC), and includes a memory. In the present embodiment, the grasping treatment instrument 2 uses heat as energy to treat a treated target. The energy operation input component 5 is, for example, a foot switch.

The grasping treatment instrument 2 includes a held unit 10 which can be held by a surgeon. The held unit 10 includes a held body 11, and the held body 11 includes a fixed handle (grip) 12 which extends across the longitudinal axis C. The held unit 10 also includes a movable handle (handle) 13 which is rotatably attached to the held body 11 and which is openable and closable relative to the fixed handle 12. The grasping treatment instrument 2 includes a cylindrical shaft (sheath) 15 which is coupled to the held body 11 from the distal side. The shaft 15 extends along the longitudinal axis C. A grasping treatment unit 20 is coupled to a distal portion of the shaft 15. The grasping treatment unit 20 includes a first grasping piece (first jaw) 21 and a second grasping piece (second jaw) 31. The first grasping piece 21 and the second grasping piece (jaw) 31 are openable and closable relative to each other.

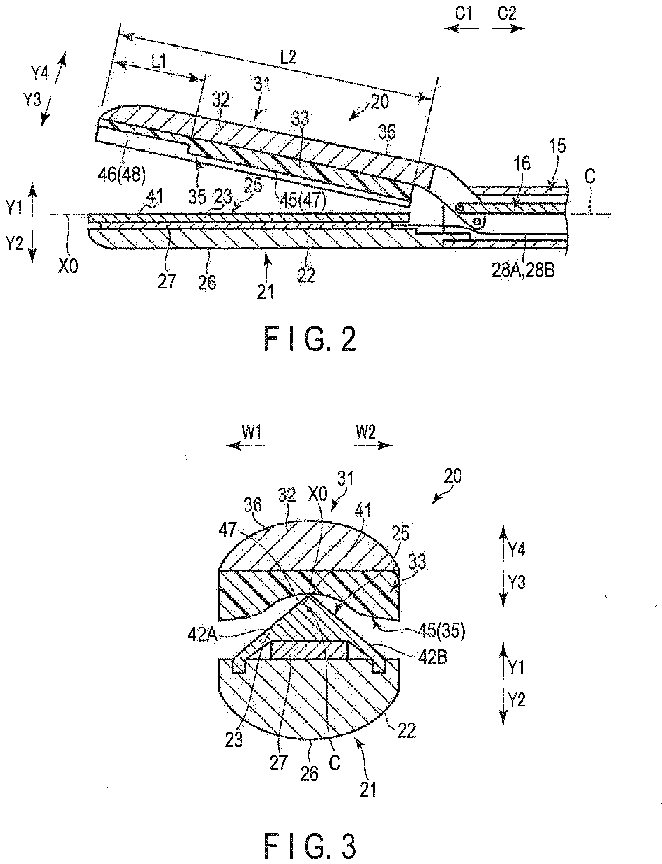

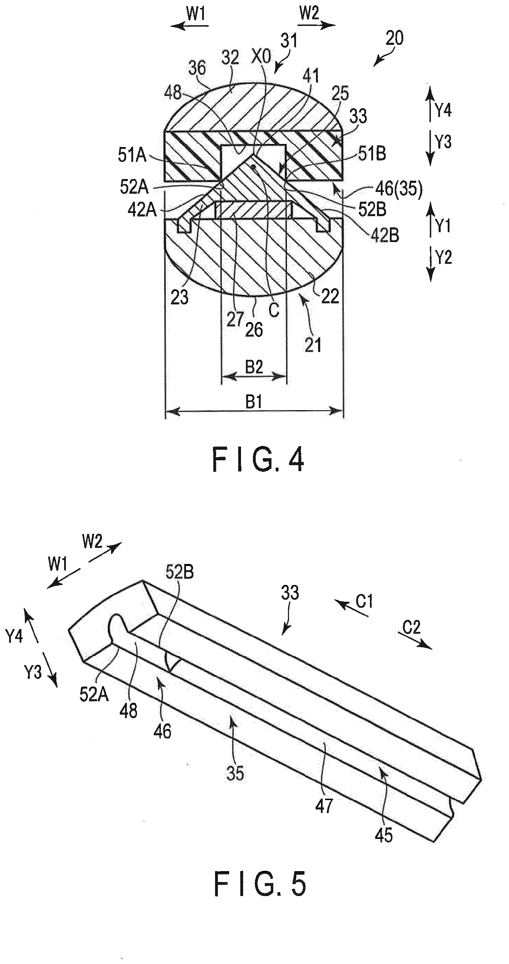

FIG. 2 is a view showing the configuration of a distal portion of the grasping treatment instrument 2 including the grasping treatment unit 20. FIG. 3 and FIG. 4 are views showing the grasping treatment unit 20 through a section perpendicular to the longitudinal axis C. FIG. 2 shows a state where the first grasping piece 21 and the second grasping piece 31 are open relative to each other, and FIG. 3 and FIG. 4 show a state where the first grasping piece 21 and the second grasping piece 31 are closed relative to each other. FIG. 3 shows a section in a proximal portion (a portion located on the proximal side with respect to a distal portion) of the grasping treatment unit 20, and FIG. 4 shows a section in the distal portion of the grasping treatment unit.

As shown in FIG. 2 to FIG. 4, the first grasping piece 21 includes a first support portion 22 fixed to the shaft 15, and a blade 23 attached to the first support portion 22. In the first grasping piece 21, the first support portion 22 and the blade 23 extend from a proximal portion to a distal portion. Here, a closing direction (a direction of an arrow Y1 in each of FIGS. 2 to 4) and an opening direction (a direction of an arrow Y2 in each of FIGS. 2 to 4) of the first grasping piece 21 which cross (is substantially perpendicular to) the longitudinal direction and which are opposite to each other are defined. In the first grasping piece 21, the blade 23 is coupled to the first support portion 22 from the closing direction side. The first support portion 22 is made of a rigid material such as a stainless alloy, and the outer surface of the first support portion 22 is coated with an electrically insulating material. The blade 23 is made of a material high in thermal conductivity, such as a copper alloy or an aluminum alloy. In the present embodiment, a first grasping surface 25 (a portion on the outer surface of the first grasping piece 21 that faces toward the closing direction side) which is a grasping surface of the first grasping piece (first jaw) 21 is formed by the blade 23, and a first back surface 26 (a portion on the outer surface of the first grasping piece 21 that faces toward the opening direction side) which is a back surface of the first grasping piece 21 is formed by the first support portion 22.

A heating body 27 such as a heater is provided between the first support portion 22 and the blade 23 in the opening and closing directions of the first grasping piece 21. The heating body 27 extends along the longitudinal direction of the first grasping piece 21 from the proximal portion to the distal portion of the first grasping piece 21, and abuts on the first support portion 22 and the blade 23. One end of each of electric wiring lines 28A and 28B is connected to the heating body 27. The electric wiring lines 28A and 28B extend through an inside of the shaft 15, an inside of the held body 11, and an inside of the cable 6, and connected at the other ends to the energy source unit 3. Heat generating electric power is supplied to the heating body 27 from the energy source unit 3 through the electric wiring lines 28A and 28B on the basis of an input of an energy operation in the energy operation input component 5. Consequently, heat generated in the heating body 27 is transmitted to the first grasping surface 25 through the blade 23. The transmitted heat is released from the first grasping surface 25 toward the second grasping piece (second jaw) 31.

The second grasping piece 31 includes a second support portion 32 rotatably attached to the shaft 15, and a pad portion 33 attached to the second support portion 32. In the second grasping piece 31, the second support portion 32 and the pad portion 33 extend from a proximal portion to a distal portion. Inside the shaft 15, a rod 16 extends along the longitudinal axis C. The rod 16 is movable relative to the shaft 15 along the longitudinal axis C. The distal portion of the rod 16 is connected to the second support portion 32. The movable handle 13 is opened or closed relative to the fixed handle 12 in the held unit 10 provided on the proximal side with respect to the grasping treatment unit 20, whereby the rod 16 moves relative to the shaft 15 along the longitudinal axis C. As a result, the second support portion 32 rotates, and the second grasping piece 31 opens or closes relative to the first grasping piece 21 (the blade 23). Accordingly, the first grasping piece 21 and the second grasping piece 31 are opened or closed relative to each other. That is, an opening/closing operation to open or close the first grasping piece 21 (the blade 23) and the second grasping piece (jaw) 31 relative to each other is input in the movable handle 13 which is an opening/closing operation input portion.

A closing direction (a direction of an arrow Y3 in each of FIGS. 2 to 4) and an opening direction (a direction of an arrow Y4 in each of FIGS. 2 to 4) of the second grasping piece 31 which cross (is substantially perpendicular to) the longitudinal direction and which are opposite to each other are also defined. In the second grasping piece 31, the pad portion 33 is coupled to the second support portion 32 from the closing direction side. The second support portion 32 is made of a rigid material such as a stainless alloy, and the outer surface of the second support portion 32 is coated with an electrically insulating material. The pad portion 33 is made of a heat-resistant and electrically insulating material such as a polytetrafluoroethylene (PTFE). In the present embodiment, a second grasping surface 35 (a portion on the outer surface of the second grasping piece 31 that faces toward the closing direction side) which is a grasping surface of the second grasping piece (second jaw) 31 is formed by the pad portion 33, and a second back surface 36 (a portion on the outer surface of the second grasping piece 31 that faces toward the opening direction side) which is a back surface of the second grasping piece 31 is formed by the second support portion 32.

The blade 23 includes a top 41 which sharpens toward the second grasping piece (jaw) 31. The top 41 extends from a proximal portion to a distal portion in the blade 23 (i.e. from the proximal portion to the distal portion of the first grasping piece 21). That is, the top 41 is continuous from the proximal portion to the distal portion of the blade 23. In the blade 23, a ridge line X0 is formed by the top 41. The top 41 is a region that includes the ridge line X0. The ridge line X0 continuously extends from the proximal portion to the distal portion of the blade 23 along the longitudinal direction of the blade 23. In the present embodiment, a portion in which the ridge line X0 extends is the apex of the top 41, and an angularity is formed at the apex of the top 41.

Here, a direction which crosses the longitudinal direction and which is substantially perpendicular to the opening and closing directions of the first grasping piece 21 (substantially perpendicular to the opening and closing directions of the second grasping piece 31) is regarded as a width direction (a direction of an arrow W1 and an arrow W2 in each of FIGS. 3 and 4). That is, the width direction crosses the longitudinal direction, and also crosses the opening and closing directions of the first grasping piece 21. In the present embodiment, the ridge line X0 extends at a substantially central position of the blade 23 (the first grasping piece 21) in the width direction. The top 41 including the ridge line X0 forms a part of the first grasping surface 25. Therefore, heat generated in the heating body 27 is transmitted to at least the top 41 through the blade 23, and released from at least the top 41 toward the second grasping piece 31.

The first grasping surface 25 formed by the blade 23 includes a first inclined plane 42A provided on one side (the arrow W1 side) of the top 41 in the width direction, and a second inclined plane 42B provided on the other side (the arrow W2 side) of the top 41 in the width direction. The first inclined plane 42A extends toward the opening direction side of the blade 23 (the first grasping piece 21) as the first inclined plane 42A comes away from the top 41 (i.e. extends toward one side of the width direction). The second inclined plane 42B extends toward the opening direction side of the blade 23 (the first grasping piece 21) as the second inclined plane 42B comes away from the top 41 (i.e. extends toward the other side of the width direction). Heat generated in the heating body 27 is transmitted to the first inclined plane 42A and the second inclined plane 42B as well as to the top 41 through the blade 23, and released toward the second grasping piece 31 from the first inclined plane 42A and the second inclined plane 42B as well.

The second grasping surface (grasping surface) 35 of the second grasping piece (second jaw) 31 extends from the proximal portion to the distal portion in the second grasping piece 31 in a state to be opposed to the top 41 of the blade 23. FIG. 5 is a view showing the configuration of the pad portion 33 of the second grasping piece 31. As shown in FIG. 2 to FIG. 5, the second grasping surface 35 is provided with a proximal side counter portion 45 which is opposed to the top 41 of the blade 23 in the proximal portion (a portion located on the proximal side with respect to the distal portion) of the second grasping piece 31, and a distal side counter portion 46 which is opposed to the top 41 of the blade 23 in the distal portion of the second grasping piece 31. That is, the proximal side counter portion 45 forms the second grasping surface 35 in the proximal portion of the second grasping piece 31 (the pad portion 33), and the distal side counter portion 46 forms the second grasping surface 35 in the distal portion of the second grasping piece 31 (the pad portion 33).

In the proximal side counter portion 45, a proximal side receiving portion (receiving portion) 47 on which the top 41 of the blade 23 can abut in a state where the second grasping piece 31 and the blade 23 (the first grasping piece 21) are closed relative to each other is formed by the pad portion 33. That is, the second grasping piece 31 is closed relative to the blade 23 in a state where the treated target is not present between the first grasping piece 21 and the second grasping piece 31, whereby the top 41 of the blade 23 abuts on the proximal side receiving portion 47 of the second grasping surface 35. On the second grasping surface (grasping surface) 35, the proximal side receiving portion 47 is formed in a state to be recessed toward the opening direction side of the second grasping piece (second jaw) 31. The proximal side receiving portion 47 extends from the proximal portion of the second grasping surface 35 (the second grasping piece 31) toward the distal side. The proximal side receiving portion 47 also extends along the longitudinal direction of the second grasping piece 31 at a substantially central position of the second grasping piece 31 (the pad portion 33) in the width direction. In a state where the top 41 of the blade 23 abuts on the proximal side receiving portion (receiving portion) 47 of the proximal side counter portion 45, the first inclined plane 42A and the second inclined plane 42B do not abut on the proximal side counter portion 45.

In the distal side counter portion 46, a recess 48 which is further recessed toward the opening direction side of the second grasping piece 31 from the proximal side receiving portion (receiving portion) 47 is formed by the pad portion 33. The recess 48 extends along the longitudinal direction of the second grasping piece 31 in the distal portion of the second grasping surface 35. The recess 48 extends at a substantially central position of the second grasping piece 31 (the pad portion 33) in the width direction. Therefore, the recess 48 extends in a state to be opposed to the distal portion of the top 41 of the blade 23. The proximal end of the recess 48 is adjacent to the distal end of the proximal side receiving portion 47 of the proximal side counter portion 45 along the longitudinal axis C. Here, in a certain example, a dimension B1 in the width direction of the second grasping piece 31 is 4 mm or more and 5 mm or less, whereas a dimension B2 of the recess 48 (the proximal side receiving portion 47) in the width direction of the second grasping piece 31 is 0.5 mm or more and 1 mm or less.

The proximal end of the recess 48 is continuous with the distal end of the proximal side receiving portion 47. The recess 48 extends from the proximal end to the distal end of the distal side counter portion 46 along the longitudinal direction. The distal end of the second grasping piece 31 is formed by the distal side counter portion 46. The recess 48 (the distal side counter portion 46) extends over an extending dimension L1 from the distal end of the second grasping piece 31 along the longitudinal direction (toward the proximal side). Here, in a certain example, the extending dimension L1 in the longitudinal direction of the recess 48 is 0.5 mm or more and 2.5 mm or less, and the entire length L2 of the pad portion 33 in the longitudinal direction of the second grasping piece 31 is about 18 mm.

In a state where the top 41 of the blade 23 abuts on the proximal side receiving portion (receiving portion) 47 of the proximal side counter portion 45, the top 41 of the blade 23 does not contact the distal side counter portion 46 including the recess 48. In the present embodiment, in a state where the top 41 of the blade 23 abuts on the proximal side receiving portion 47 of the proximal side counter portion 45, the blade 23 abuts on the distal side counter portion 46 in opening direction side abutment portions 51A and 51B which are regions different from the top 41 (portions other than the top 41). The opening direction side abutment portions 51A and 51B are located on the opening direction side of the blade 23 with respect to the top 41, and extend along the longitudinal direction in the distal portion of the blade 23. The opening direction side abutment portion 51A is located on one side of the top 41 in the width direction of the blade 23, and provided on the first inclined plane 42A. The opening direction side abutment portion 51B is located on the other side of the top 41 in the width direction of the blade 23, and provided on the second inclined plane 42B.

In a state where the opening direction side abutment portions 51A and 51B abut on the distal side counter portion 46 formed by the pad portion 33, the opening direction side abutment portion 51A abuts on a distal side receiving portion 52A, and the opening direction side abutment portion 51B abuts on a distal side receiving portion 52B. The distal side receiving portions 52A and 52B are located in the vicinity of an opening of the recess 48, and located on the closing direction side of the second grasping piece (second jaw) 31 with respect to a bottom surface of the recess 48. The distal side receiving portions 52A and 52B extend along the longitudinal direction of the second grasping piece 31 in the distal side counter portion 46 of the second grasping surface 35. Therefore, in a state where the top 41 of the blade 23 abuts on the proximal side receiving portion (receiving portion) 47 of the proximal side counter portion 45, each of the opening direction side abutment portions 51A and 51B abuts on the corresponding distal side receiving portion 52A or 52B over substantially the entire length of the distal side counter portion 46 in the longitudinal direction.

Next, functions and advantageous effects of the grasping treatment unit 20 and the grasping treatment instrument 2 according to the present embodiment are described. When a treated target such as a living tissue is treated, the grasping treatment unit 20 (the first grasping piece 21 and the second grasping piece 31) is inserted into, for example, a body, and the treated target is disposed between the first grasping piece 21 (the blade 23) and the second grasping piece (jaw) 31. The movable handle 13 is then moved to close relative to the fixed handle 12, whereby the first grasping piece 21 and the blade 23 are closed relative to each other, and the treated target is grasped between the pair of grasping pieces 21 and 31. An energy operation is input in the energy operation input component 5 in a state where the treated target is grasped. Consequently, heat generating electric power is supplied to the heating body 27 from the energy source unit 3. Heat is generated in the heating body 27 by the supply of the heat generating electric power, and the generated heat is transmitted to the first grasping surface 25. The transmitted heat is then released toward the second grasping piece 31 from the first grasping surface 25 including the top 41 through the treated target.

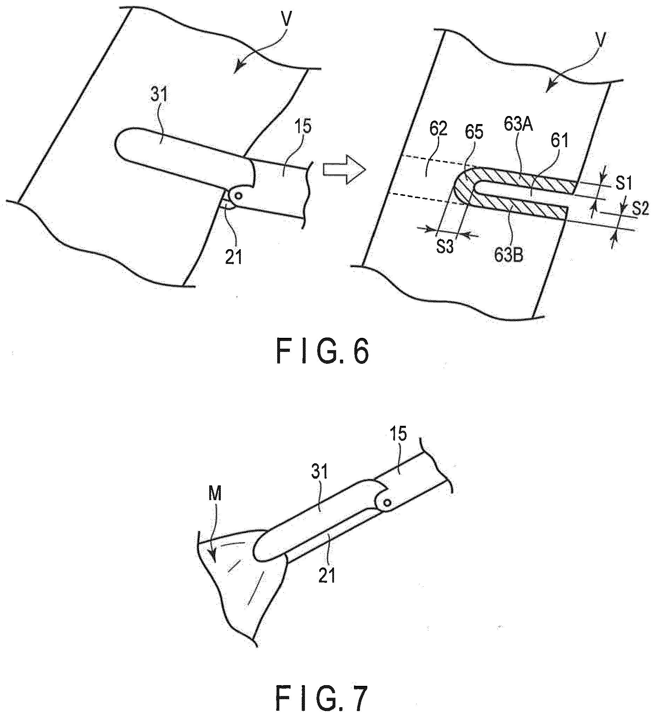

FIG. 6 is a view showing a treatment which is conducted by grasping, as a treated target, a blood vessel V having a diameter (thickness) larger than the entire length L2 of the pad portion 33 in the longitudinal direction of the second grasping piece 31. As shown in FIG. 6, the second grasping surface 35 of the second grasping piece 31 can not be brought into contact with the blood vessel V having a diameter larger than the entire length L2 of the pad portion 33 over the entire length of the blood vessel V in the diametrical direction of the blood vessel V at one time even if the blood vessel V is grasped between the first grasping piece 21 and the second grasping piece 31. Thus, in a state where the blood vessel V is grasped between the first grasping piece 21 and the second grasping piece 31, the second grasping surface 35 contacts not the entire width but only a part of the blood vessel V in the radial direction of the blood vessel V.

In a state where the treated target is not present between the first grasping piece 21 and the second grasping piece 31, the top 41 of the blade 23 abuts on the proximal side receiving portion 47 of the proximal side counter portion 45. Thus, in a state where the blood vessel V is grasped between the first grasping piece 21 and the second grasping piece 31, pressure on a region interposed between the top 41 of the blade 23 and the proximal side receiving portion 47 of the proximal side counter portion 45 in the blood vessel V is higher. Accordingly, heat is released from the first grasping surface 25 in a state where the blood vessel V is grasped between the first grasping piece 21 and the second grasping piece 31, whereby the region interposed between the top 41 of the blade 23 and the proximal side receiving portion 47 of the proximal side counter portion 45 in the blood vessel V is cut (cut open) by the pressure and the heat. As a result, a cut portion 61 is formed in the region interposed between the top 41 of the blade 23 and the proximal side receiving portion 47 of the proximal side counter portion 45 in the blood vessel V. An uncut portion 62 is also formed in a region (a region located on the distal side C1 with respect to the grasping treatment unit 20) of the blood vessel V with which the second grasping surface 35 does not come into contact in the diametrical direction of the blood vessel V.

In a state where the top 41 of the blade 23 abuts on the proximal side receiving portion (receiving portion) 47 of the proximal side counter portion 45, the first inclined plane 42A and the second inclined plane 42B are away from and do not abut on the proximal side counter portion 45. Thus, pressure on the blood vessel V between the first grasping piece 21 and the second grasping piece 31 is lower in the first inclined plane 42A located on one side of the top 41 in the width direction of the first grasping piece 21 and the second inclined plane 42B located on the other side of the top 41 in the width direction of the first grasping piece 21 than at the top 41. That is, applied pressure is lower in the region interposed between each of the first inclined plane 42A and the second inclined plane 42B and the proximal side counter portion 45 in the blood vessel V than in the region interposed between the top 41 and the proximal side receiving portion 47. Because the applied pressure is lower, the region interposed between each of the first inclined plane 42A and the second inclined plane 42B and the proximal side counter portion 45 in the blood vessel V is not cut, and is coagulated by heat. Consequently, a seal portion (width direction seal allowance) 63A or 63B in which the blood vessel V is sealed is formed on each of both sides of the cut portion 61 of the blood vessel V in the width direction of the grasping treatment unit 20 (in the extending direction of the blood vessel V). The seal portion 63A and 63B are formed, so that bleeding from both sides of the cut portion 61 is effectively prevented in the width direction of the grasping treatment unit 20 even if the blood vessel V is cut. In a certain example, the dimension B1 in the width direction of the second grasping piece 31 is 4 mm or more and 5 mm or less, whereas the dimension B2 of the top 41 and the recess 48 in the width direction of the grasping treatment unit 20 is 0.5 mm or more and 1 mm or less. In this case, a dimension (corresponding one of S1 and S2) of each of the seal portions 63A and 63B in the extending direction of the blood vessel V is, for example, 1 mm or more and 2.5 mm or less.

In a state where the top 41 of the blade 23 abuts on the proximal side receiving portion (receiving portion) 47 of the proximal side counter portion 45, the top 41 of the blade 23 does not contact the distal side counter portion 46 including the recess 48. Thus, applied pressure is lower in a region interposed between the top 41 and the distal side counter portion 46 (the recess 48) in the blood vessel V than in the region interposed between the top 41 and the proximal side receiving portion 47. Because the applied pressure is lower, the region interposed between the distal portion of the top 41 and the distal side counter portion 46 (the region in the vicinity of the place where the distal end of the top 41 of the blade 23 contacts) is not cut, and is coagulated by heat. Consequently, a seal portion (distal seal allowance) 65 in which the blood vessel V is sealed is formed between the cut portion 61 and the uncut portion 62 of the blood vessel V in the longitudinal direction of the grasping treatment unit 20 (in the diametrical direction of the blood vessel V). The seal portion 65 is formed, so that bleeding from between the place where the distal end of the top 41 of the blade 23 contacts and the uncut portion 62 is effectively prevented even if the blood vessel V is cut. That is, the region between the cut portion 61 and the uncut portion 62 in the diametrical direction of the blood vessel V can be suitably sealed. In a certain example, the extending dimension L1 in the longitudinal direction of the distal side counter portion 46 (the recess 48) is 0.5 mm or more and 2.5 mm or less. In this case, a dimension S3 of the seal portion 65 in the diametrical direction of the blood vessel V is 0.5 mm or more and 2.5 mm or less.

As described above, in the present embodiment, the seal portion (distal seal allowance) 65 is formed between the cut portion 61 and the uncut portion 62 of the blood vessel V even when the blood vessel V having a large diameter (with which the second grasping surface 35 of the second grasping piece 31 can not be brought into contact over the entire length of the blood vessel V at one time) is cut. The seal portion 65 is formed, so that in a treatment which cuts the blood vessel V having a large diameter (thickness), bleeding is prevented, and treatment performance and treatment efficiency can be ensured.

FIG. 7 is a view showing a treatment which grasps a thin membranous tissue M between the first grasping piece 21 and the second grasping piece 31. As shown in FIG. 7, when the thin membranous tissue M is grasped, the membranous tissue M is clamped between the distal portion of the blade 23 and the distal side counter portion 46 located in the distal portion of the second grasping surface 35 of the second grasping piece 31. That is, the distal portion of the blade 23 and the distal side counter portion 46 of the second grasping piece 31 alone are brought into contact with the membranous tissue M, and the membranous tissue M is not located between the proximal portion (a portion located on the proximal side with respect to the distal portion) of the blade 23 and the proximal side counter portion 45 of the second grasping piece. Here, in a state where the top 41 of the blade 23 abuts on the proximal side receiving portion (receiving portion) 47 of the proximal side counter portion 45, the top 41 of the blade 23 does not contact the distal side counter portion 46 including the recess 48. Thus, pressure on the clamped treated target is lower between the distal portion of the blade 23 and the distal side counter portion 46 (the recess 48) than between the top 41 and the proximal side receiving portion 47. However, in a state where the top 41 of the blade 23 abuts on the proximal side receiving portion 47 of the proximal side counter portion 45, the opening direction side abutment portions 51A and 51B which are portions different from the top 41 in the blade 23 abut on the distal side counter portion 46 (the corresponding distal side receiving portion 52A or 52B). Therefore, a certain degree of pressure is applied to the membranous tissue M between the distal portion of the blade 23 and the distal side counter portion 46 (the recess 48), so that the thin membranous tissue M is suitably grasped between the distal portion of the blade 23 and the distal side counter portion 46.

Therefore, in the present embodiment, even when the treated target (membranous tissue M) is grasped in a state where the distal portion of the blade 23 and the distal side counter portion 46 of the second grasping surface 35 alone are brought into contact with the treated target, a degree of pressure that can grasp a grasped target is applied to the grasped target that is clamped. Thus, the thin membranous tissue M is suitably grasped between the distal portion of the blade 23 and the distal side counter portion 46 of the second grasping surface 35, and, in a treatment in which the distal portion of the grasping treatment unit 20 alone is used to grasp the treated target, treatment efficiency and treatment performance can be ensured.

### (Modifications of First Embodiment)

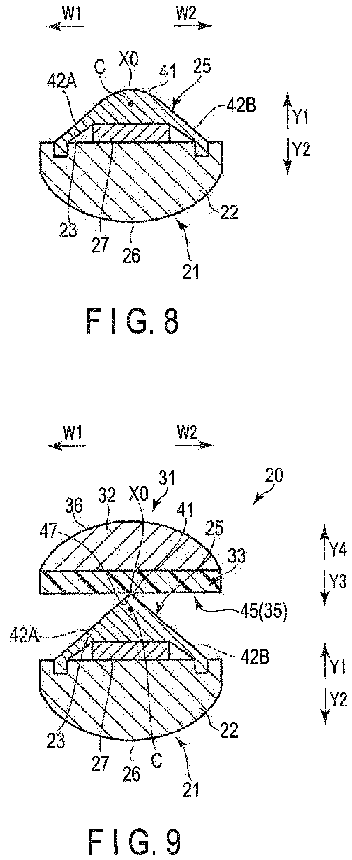

Furthermore, in the first embodiment, the angularity is formed at the apex of the top 41 where the ridge line X0 extends, but it is not limited to the above. For example, in a first modification of the first embodiment shown in FIG. 8, no angularity may be formed at the apex of the top 41, and the apex of the top 41 may be formed into a curved surface shape. In the present modification as well, the top 41 extends from the proximal portion to the distal portion of the blade 23, and the ridge line X0 is continuously formed by the top 41 from the proximal portion to the distal portion of the blade 23 along the longitudinal direction of the blade 23. Here, FIG. 8 shows a section perpendicular to the longitudinal direction of the first grasping piece 21.

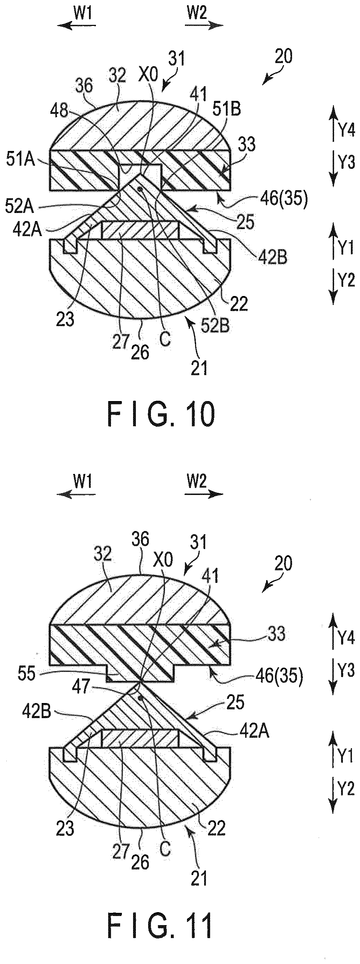

Although the proximal side receiving portion (receiving portion) 47 on which the top 41 of the blade 23 can abut is recessed toward the opening direction side of the second grasping piece (second jaw) 31 in the second grasping surface 35 in the first embodiment, it is not limited to the above. For example, in a second modification of the first embodiment shown in FIG. 9 and FIG. 10, the proximal side receiving portion 47 does not need to be recessed on the second grasping surface 35. However, in the present modification as well, the proximal end of the recess 48 is continuous with the distal end of the proximal side receiving portion 47. Here, FIG. 9 shows a section perpendicular to the longitudinal direction in the proximal portion (a portion located on the proximal side with respect to the distal portion) of the grasping treatment unit 20, and FIG. 10 shows a section perpendicular to the longitudinal direction in the distal portion of the grasping treatment unit 20. In FIG. 9 and FIG. 10, the first grasping piece 21 and the second grasping piece 31 are closed relative to each other.

In the present modification as well, in a state where the top 41 of the blade 23 abuts on the proximal side receiving portion 47, the top 41 does not contact the distal side counter portion 46 including the recess 48. Moreover, in the present modification as well, in a state where the top 41 of the blade 23 abuts on the proximal side receiving portion 47, the blade 23 abuts on the distal side counter portion 46 in the opening direction side abutment portions 51A and 51B located on the opening direction side of the blade 23 with respect to the top 41. In this instance, on the second grasping surface 35, the opening direction side abutment portion 51A abuts on the distal side receiving portion 52A, and the opening direction side abutment portion 51B abuts on the distal side receiving portion 52B.

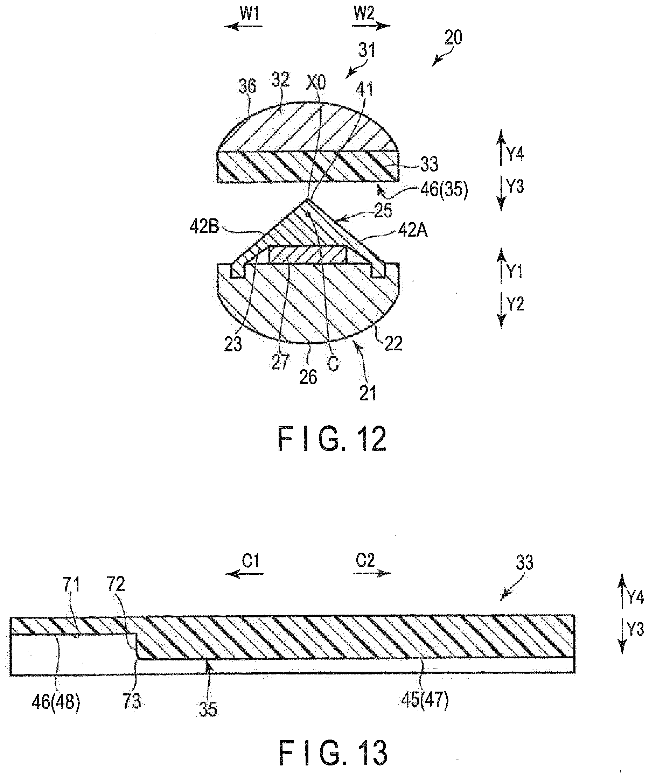

Although the recess 48 is provided in the distal side counter portion 46 in the first embodiment, it is not limited to the above. For example, in a third modification of the first embodiment shown in FIG. 11 and FIG. 12, the recess 48 does not need to be provided in the distal side counter portion 46 of the second grasping surface 35. In the present modification, a protrusion 55 which protrudes toward the closing direction side (blade 23 side) of the second grasping piece 31 is provided in the proximal side counter portion 45 of the second grasping surface 35. The protrusion 55 extends along the longitudinal direction of the second grasping piece (jaw) 31 in the proximal side counter portion 45. The proximal side receiving portion (receiving portion) 47 on which the top 41 of the blade 23 can abut is formed at a protruding end of the protrusion 55. Here, FIG. 11 shows a section perpendicular to the longitudinal direction in the proximal portion (a portion located on the proximal side with respect to the distal portion) of the grasping treatment unit 20, and FIG. 12 shows a section perpendicular to the longitudinal direction in the distal portion of the grasping treatment unit 20. In FIG. 11 and FIG. 12, the first grasping piece 21 and the second grasping piece 31 are closed relative to each other.

In the present modification, in a state where the top 41 of the blade 23 abuts on the proximal side receiving portion 47 of the protrusion 55, the distal portion of the blade 23 does not contact the distal side counter portion 46. That is, in the present modification, in a state where the top 41 of the blade 23 abuts on the proximal side receiving portion 47, the blade 23 does not contact the distal side counter portion 46 in any parts of the top 41, the first inclined plane 42A, and the second inclined plane 42B. Therefore, in the present modification, the opening direction side abutment portions 51A and 51B are not formed in the blade 23, and the distal side receiving portions 52A and 52B are not formed in the distal side counter portion 46 of the second grasping surface 35.

In a fourth modification of the first embodiment shown in FIG. 13 as well as in the first embodiment, the recess 48 is formed in the distal side counter portion 46 of the second grasping surface 35. The recess 48 includes a recess bottom surface 71, and a recess proximal surface 72 which forms the proximal end of the recess 48. In the present modification, a relay curved surface 73 in the shape of a curved surface is formed between the recess proximal surface 72 of the recess 48 and the proximal side receiving portion 47 (the proximal side counter portion 45). The relay curved surface 73 is provided so that no angularity (edge) may be formed between the recess proximal surface 72 and the proximal side receiving portion 47. This effectively prevents the top 41 of the blade 23 from abutting on the angularity (edge) on the second grasping surface 35. FIG. 13 shows the pad portion 33 through a section perpendicular to the width direction of the second grasping piece 31.

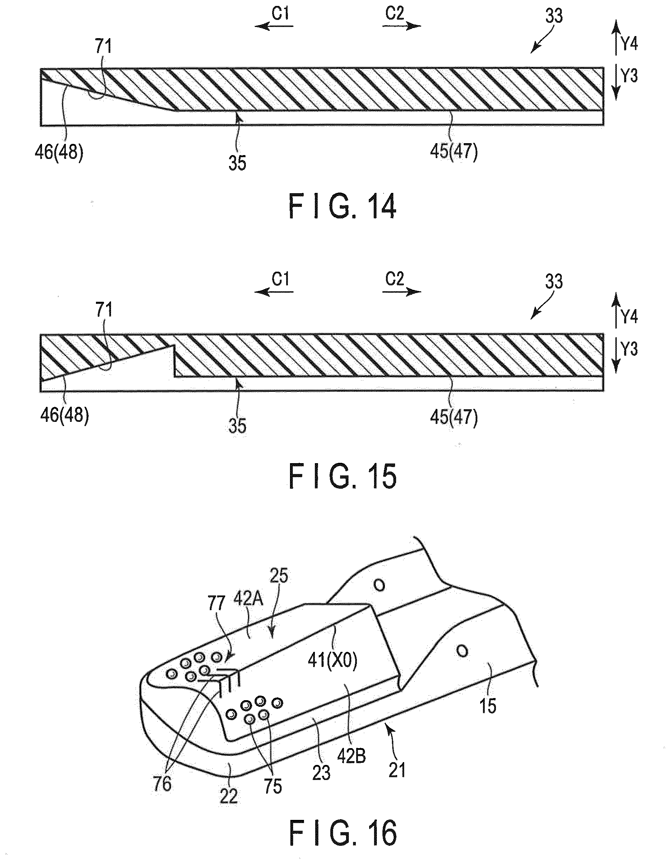

In a fifth modification of the first embodiment shown in FIG. 14, the recess 48 increases in depth toward the distal side. That is, in the present modification, the recess bottom surface 71 of the recess 48 extends toward the opening direction side of the second grasping piece 31 as extending toward the distal side, and the recess 48 is sloped. The recess 48 is formed as described above so that no angularity (edge) may be formed between the proximal end of the recess 48 and the proximal side receiving portion 47 (the proximal side counter portion 45). This effectively prevents the top 41 of the blade 23 from abutting on the angularity (edge) on the second grasping surface 35. Moreover, the recess 48 is formed as described above so that after a treated target such as the blood vessel V is cut open as described above in the first embodiment, the treated target will suitably come away from the second grasping surface 35 of the second grasping piece 31. That is, after a treated target such as the blood vessel V is cut open, close contact of the treated target with the second grasping surface 35 is effectively prevented. FIG. 14 shows the pad portion 33 through the section perpendicular to the width direction of the second grasping piece 31.

In a sixth modification of the first embodiment shown in FIG. 15, the recess 48 decreases in depth toward the distal side. That is, in the present modification, the recess bottom surface 71 of the recess 48 extends toward the closing direction side of the second grasping piece 31 as extending toward the distal side, and the recess 48 is formed into a nail shape. The recess 48 is formed as described above so that when a treated target such as the thin membranous tissue M is grasped between the distal portion of the blade 23 and the distal side counter portion 46 of the second grasping surface 35 as described above in the first embodiment, the treated target more easily comes into the recess 48. Consequently, the treated target is suitably held without slipping between the distal portion of the blade 23 and the distal side counter portion 46 of the second grasping surface 35. FIG. 15 shows the pad portion 33 through the section perpendicular to the width direction of the second grasping piece 31.

In a seventh modification of the first embodiment shown in FIG. 16, protrusions 75 and grooves 76 are provided on the first grasping surface 25 formed by the outer surface of the blade 23. The protrusions 75 and the grooves 76 are formed in the distal portion of the first grasping surface 25 (the blade 23). In the present modification, the protrusions 75 are formed on the first inclined plane 42A and the second inclined plane 42B, and the grooves 76 are formed in the top 41 and in the vicinity of the top 41. A protrusion/recess surface portion 77 is formed in the distal portion of the first grasping surface 25 by the protrusions 75 and the grooves 76. In the protrusion/recess surface portion 77, the outer surface has a protruded and recessed shape. The protrusion/recess surface portion 77 is provided in the distal portion of the blade 23 so that when a treated target such as the thin membranous tissue M is grasped between the distal portion of the blade 23 and the distal side counter portion 46 of the second grasping surface 35 as described above in the first embodiment, slipping of the treated target is effectively prevented.

In the present modification, the protrusion/recess surface portion 77 is formed from the protrusions 75 and the grooves 76. However, in a certain modification, no grooves 76 may be provided, and the protrusion/recess surface portion 77 may be formed from the protrusions 75. In another certain modification, no protrusions 75 may be provided, and the protrusion/recess surface portion 77 may be formed from the grooves 76.

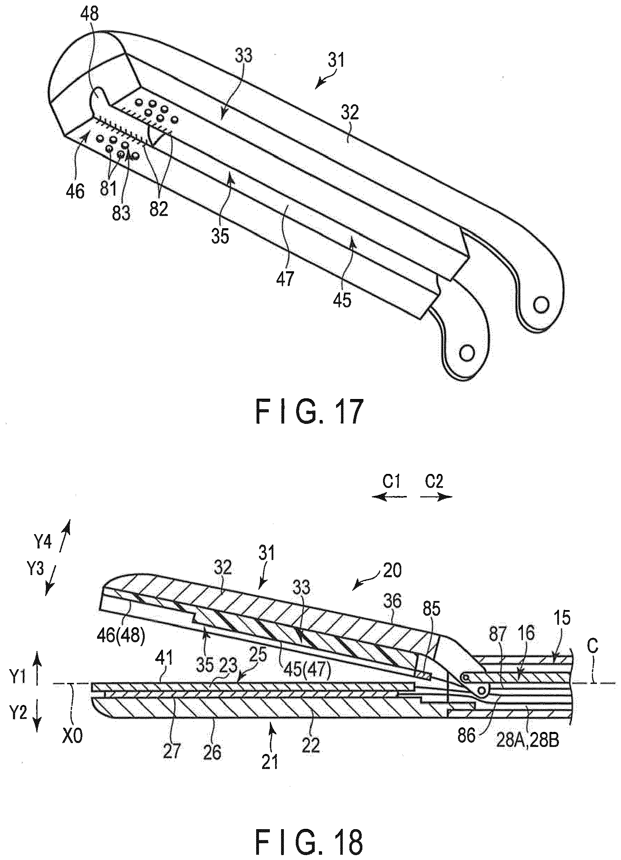

In an eighth modification of the first embodiment shown in FIG. 17, the second grasping surface 35 of the second grasping piece 31 is provided with protrusions 81 and grooves 82. The protrusions 81 and the grooves 82 are formed in the distal side counter portion 46 (distal portion) of the second grasping surface 35. In the present modification, the protrusions 81 are formed at positions away from the recess 48 in the width direction of the second grasping piece 31, and the grooves 82 are formed in the vicinity of the opening of the recess 48. A protrusion/recess surface portion 83 is formed in the distal portion (distal side counter portion 46) of the second grasping surface 35 by the protrusions 81 and the grooves 82. In the protrusion/recess surface portion 83, the outer surface has a protruded and recessed shape. The protrusion/recess surface portion 83 is provided in the distal side counter portion 46 of the second grasping surface 35 so that when a treated target such as the thin membranous tissue M is grasped between the distal portion of the blade 23 and the distal side counter portion 46 of the second grasping surface 35 as described above in the first embodiment, slipping of the treated target is effectively prevented.

In the present modification, the protrusion/recess surface portion 83 is formed from the protrusions 81 and the grooves 82. However, in a certain modification, no grooves 82 may be provided, and the protrusion/recess surface portion 83 may be formed from the protrusions 81. In another certain modification, no protrusions 81 may be provided, and the protrusion/recess surface portion 83 may be formed from the grooves 82. In yet another certain modification, the protrusion/recess surface portion 77 may be provided in the distal portion of the first grasping surface 25 of the blade 23, and the protrusion/recess surface portion 83 may be provided in the distal side counter portion 46 of the second grasping surface 35. Therefore, the protrusion/recess surface portion (77; 83; 77, 83) having an outer surface to form into a protruded and recessed shape has only to be provided in at least one of the blade 23 and the second grasping surface 35 of the second grasping piece (jaw) 31.

In yet another certain modification, in the second grasping piece 31, the pad portion 33 may be swingable relative to the second support portion 32. In this case, the pad portion 33 is attached to the second support portion 32 via a connection pin or the like, and the pad portion 33 swings (rotates) relative to the second support portion 32 around the connection pin. That is, the second grasping piece (jaw) 31 is formed into a seesaw structure.

### (Second Embodiment)

Next, a second embodiment of the present invention is described with reference to FIG. 18 to FIG. 21. The second embodiment is the following modification of the configuration of the first embodiment. The same parts as those in the first embodiment are provided with the same reference marks, and are not described.

In the present embodiment, not only heat generating electric power is output but also high-frequency electric power is output from the energy source unit 3. Therefore, the energy source unit 3 is provided with a conversion circuit which converts electric power from the electric power supply into high-frequency electric power, in addition to the conversion circuit which converts electric power from the electric power supply into heat generating electric power.

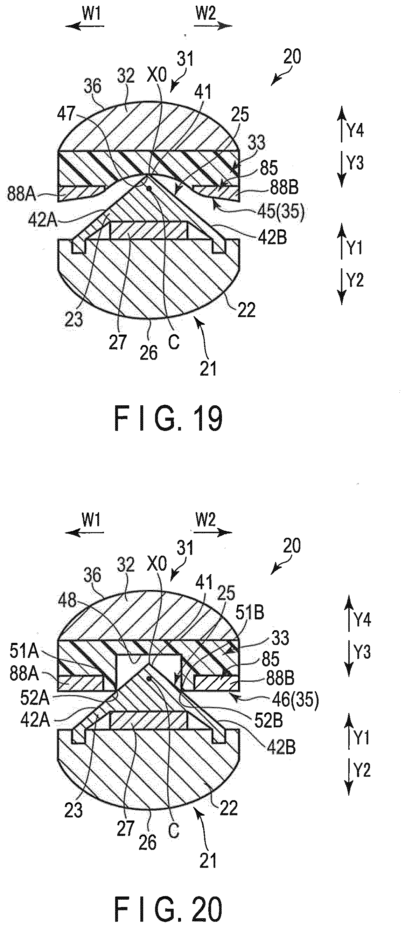

FIG. 18 is a view showing the configuration of the distal portion of the grasping treatment instrument 2 including the grasping treatment unit 20. FIG. 19 and FIG. 20 are views showing the grasping treatment unit 20 through the section perpendicular to the longitudinal axis C. FIG. 18 shows a state where the first grasping piece 21 and the second grasping piece 31 are open relative to each other, and FIG. 19 and FIG. 20 show a state where the first grasping piece 21 and the second grasping piece 31 are closed relative to each other. FIG. 19 shows a section in the proximal portion (a portion located on the proximal side with respect to the distal portion) of the grasping treatment unit 20, and FIG. 20 shows a section in the distal portion of the grasping treatment unit.

As shown in FIG. 18 to FIG. 20, in the present embodiment as well, the first grasping piece 21 includes the first support portion 22, the blade 23, and the heating body 27. In the present embodiment, the blade 23 is made of a material high in thermal conductivity, and has electric conductivity. In the present embodiment, one end of an electric wiring line 86 is connected to the blade 23. The electric wiring line 86 extends through an inside of the shaft 15, an inside of the held body 11, and an inside of the cable 6, and connected at the other end to the energy source unit 3. High-frequency electric power is supplied to the blade 23 from the energy source unit 3 through the electric wiring line 86 on the basis of an input of an energy operation in the energy operation input component 5. Since the blade 23 is made of the electrically conductive material, the blade 23 functions as an electrode by the supply of the high-frequency electric power to the blade 23. Therefore, the top 41, the first inclined plane 42A, and the second inclined plane 42B function as parts of the electrode by the supply of the high-frequency electric power to the blade 23.

In the present embodiment, a jaw electrode portion 85 is provided in the second grasping piece (jaw) 31. The jaw electrode portion 85 is made of an electrically conductive material such as a metal, and fixed to the pad portion 33. In the present embodiment, the second grasping surface (grasping surface) 35 of the second grasping piece 31 is formed by the pad portion 33 and the jaw electrode portion 85. Therefore, a part of the second grasping surface 35 is formed by the jaw electrode portion 85. One end of an electric wiring line 87 is connected to the jaw electrode portion 85. The electric wiring line 87 extends through the inside of the shaft 15, the inside of the held body 11, and the inside of the cable 6, and connected at the other end to the energy source unit 3. High-frequency electric power is supplied to the jaw electrode portion 85 from the energy source unit 3 through the electric wiring line 87 on the basis of an input of an energy operation in the energy operation input component 5. The jaw electrode portion 85 functions as an electrode different in electric potential from the blade 23 by the supply of the high-frequency electric power to the jaw electrode portion 85.

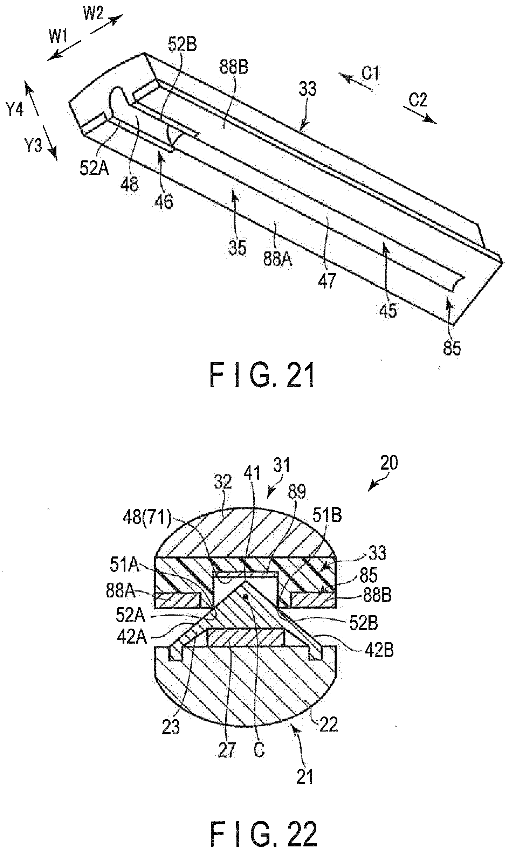

FIG. 21 is a view showing the configurations of the pad portion 33 and the jaw electrode portion 85 of the second grasping piece 31. As shown in FIG. 18 to FIG. 21, in the present embodiment as well, the second grasping surface 35 includes the proximal side counter portion 45 and the distal side counter portion 46. In the proximal side counter portion 45, the proximal side receiving portion (receiving portion) 47 is formed by the pad portion 33 made of the electrically insulating material. When the blade 23 (the first grasping piece 21) and the second grasping piece 31 are closed relative to each other, the top 41 of the blade 23 can abut on the proximal side receiving portion 47. In the present embodiment as well, the recess 48 is provided in the distal side counter portion 46 of the second grasping surface 35, and the recess 48 is continuous with the distal side of the proximal side receiving portion 47. In the present embodiment, the recess 48 is formed by the pad portion 33. Therefore, in the present embodiment, the proximal side receiving portion 47 and the recess 48 are made of the electrically insulating materials.

The jaw electrode portion 85 includes a lateral electrode portion 88A located on one side (an arrow W1 side in each of FIGS. 19 to and 21) with respect to the proximal side receiving portion 47 and the recess 48 in the width direction of the second grasping piece 31, and a lateral electrode portion 88B located on the other side (an arrow W2 side in each of FIGS. 19 to and 21) with respect to the proximal side receiving portion 47 and the recess 48 in the width direction of the second grasping piece 31. The lateral electrode portions 88A and 88B extend from the proximal portion to the distal portion of the second grasping piece 31 (the second grasping surface 35) along the longitudinal direction.

In the present modification as well, in a state where the top 41 of the blade 23 abuts on the proximal side receiving portion (receiving portion) 47 of the proximal side counter portion 45, the top 41 of the blade 23 does not contact the distal side counter portion 46 including the recess 48. Moreover, in the present modification as well, the opening direction side abutment portion 51A is provided on the first inclined plane 42A of the blade 23, and the opening direction side abutment portion 51B is provided on the second inclined plane 42B. In a state where the top 41 of the blade 23 abuts on the proximal side receiving portion 47 of the proximal side counter portion 45, at the opening direction side abutment portions 51A and 51B, the blade 23 abuts on the distal side counter portion 46. Each of the opening direction side abutment portions 51A and 51B abuts on the corresponding distal side receiving portion (52A or 52B) in the distal side counter portion 46. In the present embodiment, the distal side receiving portions 52A and 52B are formed by the pad portion 33. Therefore, the distal side receiving portions 52A and 52B are made of the electrically insulating materials.

Even if the first grasping piece 21 and the second grasping piece 31 are closed relative to each other, the blade 23 including the top 41, the first inclined plane 42A, and the second inclined plane 42B does not contact the jaw electrode portion 85. That is, the top 41 of the blade 23 can abut on the proximal side receiving portion 47 made of the electrically insulating material, and each of the opening direction side abutment portions 51A and 51B can abut on the distal side receiving portion (52A or 52B) made of the electrically insulating material, but the top 41 and the opening direction side abutment portions 51A and 51B do not contact the jaw electrode portion 85 made of the electrically conductive material.

Next, functions and advantageous effects of the grasping treatment unit 20 and the grasping treatment instrument 2 according to the present embodiment are described. In the present embodiment as well, a treated target such as the blood vessel V is grasped between the first grasping piece 21 and the second grasping piece 31, and a treatment is conducted. An energy operation is input in the energy operation input component 5 in a state where the treated target is grasped, whereby heat generating electric power is supplied to the heating body 27, and heat is generated in the heating body 27. The heat is then released to the treated target from the first grasping surface 25. Consequently, as has previously been described in the first embodiment, a treated target such as the blood vessel V is cut (cut open). The seal portion (63A, 63B, 65) is then formed in the vicinity of the cut portion 61 of the treatment target, and bleeding from the vicinity of the cut portion 61 is prevented. In this instance, functions and advantageous effects similar to those in the first embodiment described above are provided.

In the present embodiment, the energy operation is input in the energy operation input component 5, whereby high-frequency electric power is supplied to the blade 23 and the jaw electrode portion 85, and the blade 23 and the jaw electrode portion 85 function as electrodes different in electric potential from each other. The high-frequency electric power is supplied to the blade 23 and the jaw electrode portion 85 in a state where a treated target such as the blood vessel V is grasped, whereby a high-frequency electric current flows through the treated target between the blade 23 and the jaw electrode portion 85. The high-frequency electric current flows through the treated target in the vicinity of the cut portion 61, whereby the treated target is degenerated, and its coagulation is accelerated. Consequently, sealing is suitably conducted in the vicinity of the cut portion 61 of the treated target, and bleeding is effectively prevented.

Even if the first grasping piece 21 and the second grasping piece 31 are closed relative to each other, the blade 23 including the top 41, the first inclined plane 42A, and the second inclined plane 42B does not contact the jaw electrode portion 85. This prevents short circuit between the blade 23 and the jaw electrode portion 85, and prevents the high-frequency electric current from flowing between the blade 23 and the jaw electrode portion 85 without passing through the treated target. The high-frequency electric current suitably flows through the treated target between the blade 23 and the jaw electrode portion 85, whereby coagulation is suitably conducted in the vicinity of the cut portion 61 of the treated target.

### (Modifications of Second Embodiment)

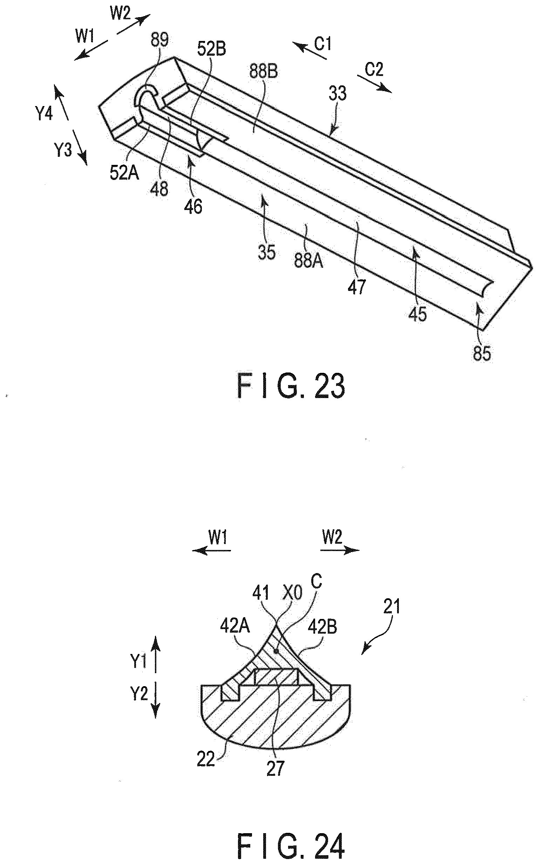

Although the whole recess 48 is formed by the pad portion 33 in the second embodiment, it is not limited to the above. For example, in a first modification of the second embodiment shown in FIG. 22 and FIG. 23, the recess 48 may be formed by the pad portion 33 and the jaw electrode portion 85 on the second grasping surface 35. In the present modification, the jaw electrode portion 85 includes a bottom surface electrode portion 89 forming the recess bottom surface 71 which is the bottom surface of the recess 48. The bottom surface electrode portion 89 extends along the longitudinal direction on the recess bottom surface 71. When supplied with the high-frequency electric power, the bottom surface electrode portion 89 has the same electric potential as in the jaw electrode portion 85. The bottom surface electrode portion 89 is exposed to the outside on the recess bottom surface 71 of the recess 48 alone, and is not exposed in regions other than the recess bottom surface 71. Therefore, in the present modification as well, the proximal side receiving portion 47 on which the top 41 can abut, and each of the distal side receiving portions 52A and 52B on which the corresponding opening direction side abutment portion 51A or 51B can abut are formed by the electrically insulating pad portion 33.

In the present modification, the bottom surface electrode portion 89 is provided in addition to the lateral electrode portions 88A and 88B, so that the exposing area of the jaw electrode portion 85 is larger on the second grasping surface 35. Accordingly, the region of the treated target to contact the jaw electrode portion 85 is larger, and the region in which the high-freguency electric current flows through the treated target between the blade 23 and the jaw electrode portion 85 is larger. Consequently, performance of coagulation of the treated target is improved, and sealing is more suitably conducted in the vicinity of the cut portion 61 of the treated target.

The configurations described above in the modifications of the first embodiment are also applicable to the configuration in which the jaw electrode portion 85 is provided as in the second embodiment. Here, in a certain modification in which the second grasping piece 31 has a seesaw structure, the pad portion 33 and the jaw electrode portion 85 are integrally swingable relative to the second support portion 32.

### (Other Modifications)

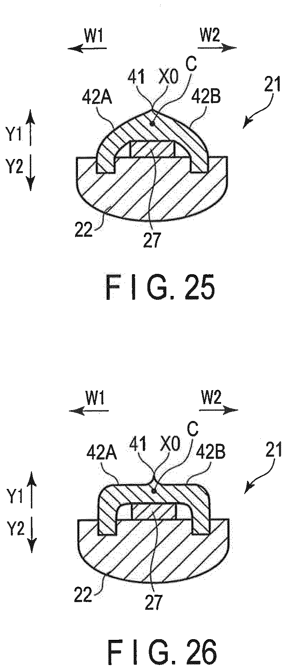

Although the first grasping surface 25 formed by the blade 23 includes the planar first inclined plane 42A provided on one side (the arrow W1 side) of the top 41 in the width direction, and the planar second inclined plane 42B provided on the other side (the arrow W2 side) of the top 41 in the width direction, the shapes of the first inclined plane 42A and the second inclined plane 42B are not limited to the above. For example, in each of modifications in FIG. 24 and FIG. 26, the first inclined plane 42A and the second inclined plane 42B are formed into curved surface shapes. In the modification in FIG. 24, in a section perpendicular to the longitudinal axis C, each of the first inclined plane 42A and the second inclined plane 42B is formed into an arc shape (curved surface shape) having its center located on the closing direction side (an arrow Y1 direction side in FIG. 24) of the first grasping piece 21 with respect to the first grasping surface 25. In the modification in FIG. 25, in the section perpendicular to the longitudinal axis C, each of the first inclined plane 42A and the second inclined plane 42B is formed into an arc shape (curved surface shape) having its center located on the opening direction side (an arrow Y2 direction side in FIG. 25) of the first grasping piece 21 with respect to the first grasping surface 25. In the modification in FIG. 26, in the section perpendicular to the longitudinal axis C, a region of each of the first inclined plane 42A and the second inclined plane 42B closer to the top 41 is formed into an arc shape (curved surface shape) having its center located on the closing direction side (an arrow Y1 direction side in FIG. 26) of the first grasping piece 21 with respect to the first grasping surface 25. Further, in the section perpendicular to the longitudinal axis C, a region of each of the first inclined plane 42A and the second inclined plane 42B farther from the top 41 is formed into an arc shape (curved surface shape) having its center located on the opening direction side (an arrow Y2 direction side in FIG. 26) of the first grasping piece 21 with respect to the first grasping surface 25.

Although the heat and the high-frequency electric current (high-frequency electric power) are shown as the energy for use in treatments in the embodiments described above, the energy for use in treatments is not limited to the above. For example, in a certain modification, the configurations described above may be applied to a grasping treatment instrument which cuts open and coagulates a treated target by use of ultrasonic vibration. The configurations described above may also be applied to a grasping treatment instrument which cuts and seals a treated target solely by pressure between the first grasping piece 21 and the second grasping piece 31 without using energy.

In the embodiments and others described above, the first grasping piece 21 including the blade 23 is fixed to the shaft 15, and the second grasping piece 31 is rotatable relative to the shaft 15, but it is not limited to the above. In a certain modification, the second grasping piece 31 may be fixed to the shaft 15, and the first grasping piece 21 may rotate relative to the shaft 15 in response to the movement of the rod 16 along the longitudinal axis C. In another certain modification, both the first grasping piece 21 and the second grasping piece 31 may rotate relative to the shaft 15 in response to the movement of the rod 16 along the longitudinal axis C, and the first grasping piece 21 and the second grasping piece 31 may open and close relative to each other.

In the embodiments and others described above, the grasping treatment unit (20) includes the blade (23) having the proximal portion and the distal portion, and the jaw (31) which has the proximal portion and the distal portion, and which is openable and closable relative to the blade (23). In the blade (23), the top (41) which sharpens toward the jaw (31) extends from the proximal portion to the distal portion, and the ridge line (X0) along the longitudinal direction of the blade (23) is formed by the top (41). In the jaw (31), the grasping surface (35) extends from the proximal portion to the distal portion in a state to be opposed to the top (41). The proximal side counter portion (45), on which the top (41) can abut in a state where the blade (23) and the jaw (31) are closed relative to each other, is provided in the region of the grasping surface (35) located on the proximal side with respect to the distal portion, and the distal side counter portion (46), which does not contact the top (41) in a state where the top (41) of the blade (23) abuts on the proximal side counter portion (45), is provided in the distal portion of the grasping surface (35).

## Claims

1. A grasping treatment unit (20) comprising:
a blade (23) having a proximal portion and a distal portion;
a jaw (31) which has a proximal portion and a distal portion, and which is openable and closable relative to the blade (23);
a top (41) which extends from the proximal portion to the distal portion in the blade (23) in a state to sharpen toward the jaw (31), and which forms a ridge line (X0) along a longitudinal direction of the blade (23); and
a grasping surface (35) which extends from the proximal portion to the distal portion in the jaw (31) in a state to be opposed to the blade (23),
wherein the grasping surface (35) includes:
a proximal side counter portion (45) which forms the grasping surface (35) in a region located on a proximal side (C2) with respect to the distal portion of the jaw (31), the proximal side counter portion (45) including a proximal side receiving portion (47) on which the top (41) can abut in a state where the blade (23) and the jaw (31) are closed relative to each other; and
a distal side counter portion (46) which forms the grasping surface (35) in the distal portion of the jaw (31), the distal side counter portion (46) including a recess (48), a proximal end of the recess (48) being adjacent to a distal end of the proximal side receiving portion (47), the top (41) and the ridge line (X0) extending toward a distal side (C1) beyond the proximal end of the recess (48) in a state in which the blade and the jaw are closed relative to each other, the top (41) being opposed to the recess (48), the recess (48) being located on an opening direction (Y4) side of the jaw (31) relative to the proximal side receiving portion (47) so that the distal side counter portion (46) does not contact the top (41) in a state where the top (41) of the blade (23) abuts on the proximal side receiving portion (47).

2. The grasping treatment unit (20) according to claim 1, **characterized in that** in the state where the top (41) abuts on the proximal side receiving portion (47) of the jaw (31), the blade (23) abuts on the distal side counter portion (46) at a position other than the top (41).

3. The grasping treatment unit (20) according to claim 2, **characterized in that** the blade (23) includes an opening direction side abutment portion (51A, 51B) which is located on an opening direction (Y2) side of the blade (23) with respect to the top (41), and which abuts on the distal side counter portion (46) in the state where the top (41) abuts on the proximal side receiving portion of the jaw (31).

4. The grasping treatment unit (20) according to claim 3, **characterized in that** when a direction which crosses the longitudinal direction (C1, C2) and which also crosses opening and closing directions (Y1, Y2) in the blade (23) is regarded as a width direction (W1, W2) of the blade (23), the blade (23) includes
a first inclined plane (42A) which is provided on one side with respect to the top (41) in the width direction (W1, W2), and which is provided in a region from the proximal portion of the blade (23) to the distal portion of the blade (23), the first inclined plane (42A) extending toward the opening direction (Y2) side of the blade (23) as the first inclined plane (42A) comes away from the top (41), the opening direction side abutment portion (51A) being located on the first inclined plane (42A), and
a second inclined plane (42B) which is provided on the other side with respect to the top (41) in the width direction (W1, W2), and which is provided in a region from the proximal portion of the blade (23) to the distal portion of the blade (23), the second inclined plane (42B) extending toward the opening direction (Y2) side of the blade (23) as the second inclined plane (42B) comes away from the top (41), the opening direction side abutment portion (51B) being located on the second inclined plane(42B), and
the top (41), the first inclined plane (42A), and the second inclined plane (42B) of the blade (23) function as at least one of a releasing part and an electrode, the releasing part releasing a heat, a high-frequency electric power is supplied to the electrode.

5. The grasping treatment unit (20) according to claim 1, **characterized in that** the blade (23) is made of an electrically conductive material, and functions as an electrode by a supply of high-frequency electric power thereto, and
the jaw (31) includes a jaw electrode portion (85) which forms a part of the grasping surface (35), and which is made of the electrically conductive material, the jaw electrode portion (85) functioning as an electrode different in electric potential from the blade (23) by the supply of the high-frequency electric power thereto.

6. The grasping treatment unit (20) according to claim 5, **characterized in that** the blade (23) includes an opening direction side abutment portion (51A, 51B) which is located on an opening direction (Y2) side of the blade (32) with respect to the top (41), and which abuts on the distal side counter portion (46) in the state where the top (41) abuts on the proximal side receiving portion (47) of the jaw (31),
the proximal side receiving portion (47) is made of an electrically insulating material,
the distal side counter portion (46) includes a distal side receiving portion (52A, 52B) which is made of an electrically insulating material, and on which the opening direction side abutment portion (51A,51B) abuts in the state where the top (41)abuts on the proximal side receiving portion (47), and
the top (41) and the opening direction side abutment portion (51A, 51B) do not contact the jaw electrode portion (85).

7. The grasping treatment unit (20) according to claim 6, **characterized in that** the recess (48) is recessed toward the opening direction (Y4) side of the jaw (31) relative to the proximal side receiving portion (47), and
the jaw electrode portion (85) includes a bottom surface electrode portion (89) which forms a bottom surface of the recess (48).

8. The grasping treatment unit (20) according to claim 7, **characterized in that** when supplied with the high-frequency electric power, the bottom surface electrode portion (89) has the same electric potential as in the jaw electrode portion (85).

9. The grasping treatment unit (20) according to claim 5, **characterized in that** when a direction which crosses the longitudinal direction (C1, C2) and which also crosses opening and closing directions (Y1, Y2) in the blade (23) is regarded as a width direction (W1, W2) of the blade (23), the blade (23) includes
a first inclined plane (42A) which is provided on one side with respect to the top (41) in the width direction (W1, W2), and which extends toward an opening direction (Y2) side of the blade (23) as the first inclined plane (42A) comes away from the top (41), the first inclined plane (42A) becoming a part of the electrode when supplied with the high-frequency electric power, and
a second inclined plane (42B) which is provided on the other side with respect to the top (41) in the width direction (W1, W2), and which extends toward the opening direction (Y2) side of the blade (23) as the second inclined plane (42B) comes away from the top (41), the second inclined plane (42B) becoming a part of the electrode when supplied with the high-frequency electric power.

10. The grasping treatment unit (20) according to claim 1, **characterized in that** the blade (23) releases heat from at least the top (41) toward the jaw (31).

11. The grasping treatment unit (20) according to claim 10, **characterized by** comprising a heating body (27) disposed in the blade (23).

12. The grasping treatment unit (20) according to claim 10, **characterized in that** when a direction which crosses the longitudinal direction (C1, C2) and which also crosses opening and closing directions (Y1, Y2) in the blade (23) is regarded as a width direction (W1, W2) of the blade (23), the blade (23) includes
a first inclined plane (42A) which is provided on one side with respect to the top (41) in the width direction (W1, W2), and which extends toward an opening direction (Y2) side of the blade (23) as the first inclined plane (42A) comes away from the top (41), the first inclined plane (42A) releasing the heat toward the jaw (31), and
a second inclined plane (42B) which is provided on the other side with respect to the top (41) in the width direction (W1,W2), and which extends toward the opening direction (Y2) side of the blade (23) as the second inclined plane (42B) comes away from the top (41), the second inclined plane (42B) releasing the heat toward the jaw (31).

13. The grasping treatment unit (20) according to claim 1, **characterized in that** the recess (48) is recessed toward the opening direction (Y4) side of the jaw (31) relative to the proximal side receiving portion (47).

14. The grasping treatment unit (20) according to claim 13, **characterized in that** the proximal side receiving portion (47) is recessed toward the opening direction (Y4) side of the jaw (31) on the grasping surface (35), and
the recess (48) is further recessed toward the opening direction (Y4) side of the jaw (31) relative to the proximal side receiving portion (47).

15. The grasping treatment unit (20) according to claim 1, **characterized in that** the proximal side receiving portion (47) protrudes toward a closing direction (Y3)side of the jaw (31) on the grasping surface (35).

16. The grasping treatment unit (20) according to claim 1, **characterized in that** the distal side counter portion (46) forms a distal end of the jaw (31), and extends over a dimension of 0.5 mm or more and 2.5 mm or less from the distal end of the jaw (31) in a longitudinal direction (C1, C2).

## Patentansprüche

1. Greifbehandlungseinheit (20), umfassend:
eine Klinge (23) mit einem proximalen Abschnitt und einem distalen Abschnitt;
eine Backe (31), die einen proximalen Abschnitt und einen distalen Abschnitt aufweist und die in Bezug auf die Klinge (23) öffenbar und schließbar ist;
einen Oberteil (41), der sich von dem proximalen Abschnitt zu dem distalen Abschnitt in der Klinge (23) in einem sich zuspitzenden Zustand hin zu der Backe (31) erstreckt und der eine Gratlinie (X0) entlang einer Längsrichtung der Klinge (23) bildet; und
eine Greiffläche (35), die sich von dem proximalen Abschnitt zu dem distalen Abschnitt in der Backe (31) in einem entgegengesetzten Zustand zu der Klinge (23) erstreckt,
wobei die Greiffläche (35) umfasst:
einen Gegenabschnitt (45) an einer proximalen Seite, der die Greiffläche (35) in einem sich an einer proximalen Seite (C2) in Bezug auf den distalen Abschnitt der Backe (31) befindenden Bereich bildet, wobei der Gegenabschnitt (45) an der proximalen Seite einen Aufnahmeabschnitt (47) an der proximalen Seite umfasst, auf dem der Oberteil (41) in einem Zustand, in dem die Klinge (23) und die Backe (31) relativ zueinander geschlossen sind, aufliegen kann; und
einen Gegenabschnitt (46) an einer distalen Seite, der die Greiffläche (35) in dem distalen Abschnitt der Backe (31) bildet, wobei der Gegenabschnitt (46) an der distalen Seite einen Rückstand (48) umfasst, wobei ein proximales Ende des Rückstands (48) zu einem distalen Ende des Aufnahmeabschnitts (47) an der proximalen Seite benachbart ist, wobei sich der Oberteil (41) und die Gratlinie (X0) in einem Zustand, in dem die Klinge und die Backe relativ zueinander geschlossen sind, hin zu einer distalen Seite (C1) über das proximale Ende des Rückstands (48) hinaus erstrecken, wobei der Oberteil (41) zu dem Rückstand (48) entgegengesetzt ist, wobei sich der Rückstand (48) an einer Seite einer Öffnungsrichtung (Y4) der Backe (31) in Bezug auf den Aufnahmeabschnitt (47) an der proximalen Seite befindet, sodass der Gegenabschnitt (46) an der distalen Seite in einem Zustand, in dem der Oberteil (41) der Klinge (23) auf dem Aufnahmeteil (47) an der proximalen Seite aufliegt, nicht mit dem Oberteil (41) in Kontakt steht.

2. Greifbehandlungseinheit (20) nach Anspruch 1, **dadurch gekennzeichnet, dass** in dem Zustand, in dem der Oberteil (41) auf dem Aufnahmeabschnitt (47) an der proximalen Seite der Backe (31) aufliegt, die Klinge (23) auf dem Gegenabschnitt (46) der distalen Seite an einer anderen Position als der Oberteil (41) aufliegt.

3. Greifbehandlungseinheit (20) nach Anspruch 2, **dadurch gekennzeichnet, dass** die Klinge (23) einen Auflageabschnitt (51A, 51B) an einer Seite einer Öffnungsrichtung umfasst, der sich an einer Seite einer Öffnungsrichtung (Y2) der Klinge (23) in Bezug auf den Oberteil (41) befindet und der in dem Zustand, in dem der Oberteil (41) auf dem Aufnahmeabschnitt an der proximalen Seite der Backe (31) aufliegt, auf dem Gegenabschnitt (46) der distalen Seite aufliegt.

4. Greifbehandlungseinheit (20) nach Anspruch 3, **dadurch gekennzeichnet, dass**, wenn eine Richtung, die die Längsrichtung (C1, C2) kreuzt und die auch die Öffnungs- und die Schließrichtung (Y1, Y2) in der Klinge (23) kreuzt, als eine Breitenrichtung (W1, W2) der Klinge (23) angesehen wird, die Klinge (23) umfasst:
eine erste geneigte Ebene (42A), die an einer Seite in Bezug auf den Oberteil (41) in der Breitenrichtung (W1, W2) vorgesehen ist und die in einem Bereich von dem proximalen Abschnitt der Klinge (23) zu dem distalen Abschnitt der Klinge (23) vorgesehen ist, wobei sich die erste geneigte Ebene (42A) hin zu der Seite der Öffnungsrichtung (Y2) der Klinge (23) erstreckt, wenn sich die erste geneigte Ebene (42A) von dem Oberteil (41) weg bewegt, wobei sich der Auflageabschnitt (51A) an der Seite der Öffnungsrichtung auf der ersten geneigten Ebene (42A) befindet, und
eine zweite geneigte Ebene (42B), die an der anderen Seite in Bezug auf den Oberteil (41) in der Breitenrichtung (W1, W2) vorgesehen ist und die in einem Bereich von dem proximalen Abschnitt der Klinge (23) zu dem distalen Abschnitt der Klinge (23) vorgesehen ist, wobei sich die zweite geneigte Ebene (42B) hin zu der Seite der Öffnungsrichtung (Y2) der Klinge (23) erstreckt, wenn sich die zweite geneigte Ebene (42B) von dem Oberteil (41) weg bewegt, wobei sich der Auflageabschnitt (51B) an der Seite der Öffnungsrichtung auf der zweiten geneigten Ebene (42B) befindet, und
der Oberteil (41), die erste geneigte Ebene (42A) und die zweite geneigte Ebene (42B) als zumindest eines von einem Freisetzungsteil und einer Elektrode fungieren, wobei der Freisetzungsteil eine Wärme freisetzt und der Elektrode eine elektrische Energie mit hoher Frequenz zugeführt wird.

5. Greifbehandlungseinheit (20) nach Anspruch 1, **dadurch gekennzeichnet, dass** die Klinge (23) aus einem elektrisch leitenden Material hergestellt ist und durch eine Zufuhr von elektrischer Energie mit hoher Frequenz daran als eine Elektrode fungiert, und
die Backe (31) einen Backenelektrodenabschnitt (85) umfasst, der einen Teil der Greiffläche (35) bildet und der aus dem elektrisch leitenden Material hergestellt ist, wobei der Backenelektrodenabschnitt (85) durch die Zufuhr der elektrischen Energie mit hoher Frequenz daran als eine Elektrode mit einem von der Klinge (23) unterschiedlichen elektrischen Potenzial fungiert.

6. Greifbehandlungseinheit (20) nach Anspruch 5, **dadurch gekennzeichnet, dass** die Klinge (23) einen Auflageabschnitt (51A, 51B) an einer Seite einer Öffnungsrichtung umfasst, der sich an einer Seite einer Öffnungsrichtung (Y2) der Klinge (23) in Bezug auf den Oberteil (41) befindet und der in dem Zustand, in dem der Oberteil (41) auf dem Aufnahmeabschnitt (47) an der proximalen Seite der Backe (31) aufliegt, auf dem Gegenabschnitt (46) der distalen Seite aufliegt,
der Aufnahmeabschnitt (47) an der proximalen Seite aus einem elektrisch isolierenden Material hergestellt ist,
der Gegenabschnitt (46) an der distalen Seite einen Aufnahmeabschnitt (52A, 52B) an der distalen Seite umfasst, der aus einem elektrisch isolierenden Material hergestellt ist und auf dem der Auflageabschnitt (51A, 51B) an der Seite der Öffnungsrichtung in dem Zustand aufliegt, in dem der Oberteil (41) auf dem Aufnahmeabschnitt (47) an der proximalen Seite aufliegt, und
der Oberteil (41) und der Auflageabschnitt (51A, 51B) an der Seite der Öffnungsrichtung mit dem Backenelektrodenabschnitt (85) nicht in Kontakt stehen.

7. Greifbehandlungseinheit (20) nach Anspruch 6, **dadurch gekennzeichnet, dass** der Rückstand (48) hin zu der Seite der Öffnungsrichtung (Y4) der Backe (31) in Bezug auf den Aufnahmeabschnitt (47) an der proximalen Seite zurückgesetzt ist, und
der Backenelektrodenabschnitt (85) einen Bodenflächenelektrodenabschnitt (89) umfasst, der eine Bodenfläche des Rückstands (48) bildet.

8. Greifbehandlungseinheit (20) nach Anspruch 7, **dadurch gekennzeichnet, dass** der Bodenflächenelektrodenabschnitt (89), wenn ihm eine elektrische Energie mit hoher Frequenz zugeführt wird, das gleiche elektrische Potenzial wie der Backenelektrodenabschnitt (85) aufweist.

9. Greifbehandlungseinheit (20) nach Anspruch 5, **dadurch gekennzeichnet, dass**, wenn eine Richtung, die die Längsrichtung (C1, C2) kreuzt und die auch die Öffnungs- und die Schließrichtung (Y1, Y2) in der Klinge (23) kreuzt, als eine Breitenrichtung (W1, W2) der Klinge (23) angesehen wird, die Klinge (23) umfasst:
eine erste geneigte Ebene (42A), die an einer Seite in Bezug auf den Oberteil (41) in der Breitenrichtung (W1, W2) vorgesehen ist und die sich hin zu einer Seite einer Öffnungsrichtung (Y2) der Klinge (23) erstreckt, wenn sich die erste geneigte Ebene (42A) von dem Oberteil (41) weg bewegt, wobei die erste geneigte Ebene (42A) zu einem Teil der Elektrode wird, wenn ihr die elektrische Energie mit hoher Frequenz zugeführt wird, und
eine zweite geneigte Ebene (42B), die an der anderen Seite in Bezug auf den Oberteil (41) in der Breitenrichtung (W1, W2) vorgesehen ist und die sich hin zu der Seite der Öffnungsrichtung (Y2) der Klinge (23) erstreckt, wenn sich die zweite geneigte Ebene (42B) von dem Oberteil (41) weg bewegt, wobei die zweite geneigte Ebene (42B) zu einem Teil der Elektrode wird, wenn ihr die elektrische Energie mit hoher Frequenz zugeführt wird.

10. Greifbehandlungseinheit (20) nach Anspruch 1, **dadurch gekennzeichnet, dass** die Klinge (23) Wärme zumindest von dem Oberteil (41) hin zu der Backe (31) freisetzt.

11. Greifbehandlungseinheit (20) nach Anspruch 10, **dadurch gekennzeichnet, dass** sie einen in der Klinge (23) angeordneten Heizkörper (27) umfasst.

12. Greifbehandlungseinheit (20) nach Anspruch 10, **dadurch gekennzeichnet, dass**, wenn eine Richtung, die die Längsrichtung (C1, C2) kreuzt und die auch die Öffnungs- und die Schließrichtung (Y1, Y2) in der Klinge (23) kreuzt, als eine Breitenrichtung (W1, W2) der Klinge (23) angesehen wird, die Klinge (23) umfasst:
eine erste geneigte Ebene (42A), die an einer Seite in Bezug auf den Oberteil (41) in der Breitenrichtung (W1, W2) vorgesehen ist und die sich hin zu einer Seite einer Öffnungsrichtung (Y2) der Klinge (23) erstreckt, wenn sich die erste geneigte Ebene (42A) von dem Oberteil (41) weg bewegt, wobei die erste geneigte Ebene (42A) die Wärme hin zu der Backe (31) freisetzt, und
eine zweite geneigte Ebene (42B), die an der anderen Seite in Bezug auf den Oberteil (41) in der Breitenrichtung (W1, W2) vorgesehen ist und die sich hin zu der Seite der Öffnungsrichtung (Y2) der Klinge (23) erstreckt, wenn sich die zweite geneigte Ebene (42B) von dem Oberteil (41) weg bewegt, wobei die zweite geneigte Ebene (42B) die Wärme hin zu der Backe (31) freisetzt.

13. Greifbehandlungseinheit (20) nach Anspruch 1, **dadurch gekennzeichnet, dass** der Rückstand (48) hin zu der Seite der Öffnungsrichtung (Y4) der Backe (31) in Bezug auf den Aufnahmeabschnitt (47) an der proximalen Seite zurückgesetzt ist.

14. Greifbehandlungseinheit (20) nach Anspruch 13, **dadurch gekennzeichnet, dass** der Aufnahmeabschnitt (47) an der proximalen Seite hin zu der Seite der Öffnungsrichtung (Y4) der Backe (31) an der Greiffläche (35) zurückgesetzt ist, und
der Rückstand (48) ferner hin zu der Seite der Öffnungsrichtung (Y4) der Backe (31) in Bezug auf den Aufnahmeabschnitt (47) an der proximalen Seite zurückgesetzt ist.

15. Greifbehandlungseinheit (20) nach Anspruch 1, **dadurch gekennzeichnet, dass** der Aufnahmeabschnitt (47) an der proximalen Seite hin zu der Seite einer Schließrichtung (Y3) der Backe (31) an der Greiffläche (35) vorsteht.

16. Greifbehandlungseinheit (20) nach Anspruch 1, **dadurch gekennzeichnet, dass** der Gegenabschnitt (46) an der distalen Seite ein distales Ende der Backe (31) bildet und sich über eine Abmessung von 0,5 mm oder mehr und 2,5 mm oder weniger von dem distalen Ende der Backe (31) in einer Längsrichtung (C1, C2) erstreckt.

## Revendications

1. Unité de traitement de préhension (20) comprenant :
une lame (23) ayant une partie proximale et une partie distale ;
une mâchoire (31) qui a une partie proximale et une partie distale, et qui est apte à s'ouvrir et se fermer par rapport à la lame (23) ;
une partie supérieure (41) qui s'étend de la partie proximale à la partie distale dans la lame (23) dans un état s'effilant vers la mâchoire (31), et qui forme une ligne d'arête (X0) le long d'une direction longitudinale de la lame (23) ; et
une surface de préhension (35) s'étend de la partie proximale à la partie distale dans la mâchoire (31) dans un état de façon à être opposé à la lame (23),
la surface de préhension (35) comprenant :
une contre-partie côté proximal (45) qui forme la surface de préhension (35) dans une région située sur un côté proximal (C2) par rapport à la partie distale de la mâchoire (31), la contre-partie côté proximal (45) comprenant une partie de réception côté proximal (47) sur laquelle la partie supérieure (41) peut venir en butée dans un état dans lequel la lame (23) et la mâchoire (31) sont fermées l'une par rapport à l'autre ; et
une contre-partie côté distal (46) qui forme la surface de préhension (35) dans la partie distale de la mâchoire (31), la contre-partie côté distal (46) comprenant un évidement (48), une extrémité proximale de l'évidement (48) étant adjacente à une extrémité distale de la partie de réception côté proximal (47), la partie supérieure (41) et la ligne d'arête (X0) s'étendant vers un côté distal (C1) au-delà de l'extrémité proximale de l'évidement (48) dans un état dans lequel la lame et la mâchoire sont fermées l'une par rapport à l'autre, la partie supérieure (41) étant opposée à l'évidement (48), l'évidement (48) étant situé sur un côté direction d'ouverture (Y4) de la mâchoire (31) par rapport à la partie de réception côté proximal (47) de telle sorte que la contre-partie côté distal (46) n'est pas en contact avec la partie supérieure (41) dans un état dans lequel la partie supérieure (41) de la lame (23) est en butée sur la partie de réception côté proximal (47).

2. Unité de traitement de préhension (20) selon la revendication 1, **caractérisée par le fait que** dans l'état dans lequel la partie supérieure (41) est en butée sur la partie de réception côté proximal (47) de la mâchoire (31), la lame (23) est en butée sur la contre-partie côté distal (46) dans une position autre que la partie supérieure (41).

3. Unité de traitement de préhension (20) selon la revendication 2, **caractérisée par le fait que** la lame (23) comprend une partie de butée côté direction d'ouverture (51A, 51B) qui est située sur un côté direction d'ouverture (Y2) de la lame (23) par rapport à la partie supérieure (41), et qui s'appuie sur la contre-partie côté distal (46) dans l'état dans lequel la partie supérieure (41) s'appuie sur la partie de réception côté proximal de la mâchoire (31).

4. Unité de traitement de préhension (20) selon la revendication 3, **caractérisée par le fait que**, lorsqu'une direction qui coupe la direction longitudinale (C1, C2) et qui coupe également des directions d'ouverture et de fermeture (Y1, Y2) dans la lame (3) est considérée comme direction de largeur (W1, W2) de la lame (23), la lame (23) comprend
un premier plan incliné (42A) qui se trouve sur un côté par rapport à la partie supérieure (41) dans la direction de largeur (W1, W2), et qui se trouve dans une région allant de la partie proximale de la lame (23) à la partie distale de la lame (23), le premier plan incliné (42A) s'étendant vers le côté direction d'ouverture (Y2) de la lame (23) à mesure que le premier plan incliné (42A) s'éloigne de la partie supérieure (41), la partie de butée côté direction d'ouverture (51A) étant située sur le premier plan incliné (42A), et
un second plan incliné (42B) qui se trouve sur l'autre côté par rapport à la partie supérieure (41) dans la direction de largeur (W1, W2), et qui se trouve dans une région allant de la partie proximale de la lame (23) à la partie distale de la lame (23), le second plan incliné (42B) s'étendant vers le côté direction d'ouverture (Y2) de la lame (23) à mesure que le second plan incliné (42B) s'éloigne de la partie supérieure (41), la partie de butée côté direction d'ouverture (51B) étant située sur le second plan incliné (42B), et
la partie supérieure (41), le premier plan incliné (42A) et le second plan incliné (42B) de la lame (23) agissent en tant qu'au moins une parmi une partie de libération et une électrode, la partie de libération libérant de la chaleur, une puissance électrique haute fréquence étant fournie à l'électrode.

5. Unité de traitement de préhension (20) selon la revendication 1, **caractérisée par le fait que** la lame (23) est faite d'un matériau électroconducteur et agit en tant qu'électrode par une fourniture de puissance électrique haute fréquence à celle-ci, et
la mâchoire (31) comprend une partie électrode de mâchoire (85) qui fait partie de la surface de préhension (35) et qui est faite du matériau électroconducteur, la partie électrode de mâchoire (85) agissant en tant qu'électrode qui diffère en potentiel électrique de la lame (23) par la fourniture de la puissance électrique haute fréquence à celle-ci.

6. Unité de traitement de préhension (20) selon la revendication 5, **caractérisée par le fait que** la lame (23) comprend une partie de butée côté direction d'ouverture (51A, 51B) qui est située sur un côté direction d'ouverture (Y2) de la lame (23) par rapport à la partie supérieure (41), et qui est en butée sur la contre-partie côté distal (46) dans l'état dans lequel la partie supérieure (41) est en butée sur la partie de réception côté proximal (47) de la mâchoire (31),
la partie de réception côté proximal (47) est faite d'un matériau électriquement isolant,
la contre-partie côté distal (46) comprend une partie de réception côté distal (52A, 52B) qui est faite d'un matériau électriquement isolant, et sur laquelle la partie de butée côté direction d'ouverture (51A, 51B) est en butée dans l'état dans lequel la partie supérieure (41) est en butée sur la partie de réception côté proximal (47), et
la partie supérieure (41) et la partie de butée côté direction d'ouverture (51A, 51B) n'entrent pas en contact avec la partie électrode de mâchoire (85).

7. Unité de traitement de préhension (20) selon la revendication 6, **caractérisée par le fait que** l'évidement (48) est en retrait vers le côté direction d'ouverture (Y4) de la mâchoire (31) par rapport à la partie de réception côté proximal (47), et
la partie électrode de mâchoire (85) comprend une partie électrode de surface inférieure (89) qui forme une surface inférieure de l'évidement (48).

8. Unité de traitement de préhension (20) selon la revendication 7, **caractérisée par le fait que**, lorsqu'elle reçoit la puissance électrique haute fréquence, la partie électrode de surface inférieure (89) a le même potentiel électrique que la partie électrode de mâchoire (85).

9. Unité de traitement de préhension (20) selon la revendication 5, **caractérisée par le fait que**, lorsqu'une direction qui coupe la direction longitudinale (C1, C2) et qui coupe également des directions d'ouverture et de fermeture (Y1, Y2) dans la lame (23) est considérée comme direction de largeur (W1, W2) de la lame (23), la lame (23) comprend
un premier plan incliné (42A) qui se trouve sur un côté par rapport à la partie supérieure (41) dans la direction de largeur (W1, W2), et qui s'étend vers un côté direction d'ouverture (Y2) de la lame (23) à mesure que le premier plan incliné (42A) s'éloigne de la partie supérieure (41), le premier plan incliné (42A) devenant une partie de l'électrode lorsque la puissance électrique haute fréquence lui est fournie, et
un second plan incliné (42B) qui se trouve sur l'autre côté par rapport à la partie supérieure (41) dans la direction de largeur (W1, W2), et qui s'étend vers le côté direction d'ouverture (Y2) de la lame (23) à mesure que le second plan incliné (42B) s'éloigne de la partie supérieure (41), le second plan incliné (42B) devenant une partie de l'électrode lorsque la puissance électrique haute fréquence lui est fournie.

10. Unité de traitement de préhension (20) selon la revendication 1, **caractérisée par le fait que** la lame (23) libère de la chaleur au moins depuis la partie supérieure (41) vers la mâchoire (31).

11. Unité de traitement de préhension (20) selon la revendication 10, **caractérisée par le fait qu'**elle comprend un corps chauffant (27) disposé dans la lame (23).

12. Unité de traitement de préhension (20) selon la revendication 10, **caractérisée par le fait que**, lorsqu'une direction qui coupe la direction longitudinale (C1, C2) et qui coupe également des directions d'ouverture et de fermeture (Y1, Y2) dans la lame (23) est considérée comme direction de largeur (W1, W2) de la lame (23), la lame (23) comprend
un premier plan incliné (42A) qui se trouve sur un côté par rapport à la partie supérieure (41) dans la direction de largeur (W1, W2), et qui s'étend vers un côté direction d'ouverture (Y2) de la lame (23) à mesure que le premier plan incliné (42A) s'éloigne de la partie supérieure (41), le premier plan incliné (42A) libérant la chaleur vers la mâchoire (31), et
un second plan incliné (42B) qui se trouve sur l'autre côté par rapport à la partie supérieure (41) dans la direction de largeur (W1, W2), et qui s'étend vers le côté direction d'ouverture (Y2) de la lame (23) à mesure que le second plan incliné (42B) s'éloigne de la partie supérieure (41), le second plan incliné (42B) libérant la chaleur vers la mâchoire (31).

13. Unité de traitement de préhension (20) selon la revendication 1, **caractérisée par le fait que** l'évidement (48) est en retrait vers le côté direction d'ouverture (Y4) de la mâchoire (31) par rapport à la partie de réception côté proximal (47).

14. Unité de traitement de préhension (20) selon la revendication 13, **caractérisée par le fait que** la partie de réception côté proximal (47) est en retrait vers le côté direction d'ouverture (Y4) de la mâchoire (31) sur la surface de préhension (35), et
l'évidement (48) est en outre en retrait vers le côté direction d'ouverture (Y4) de la mâchoire (31) par rapport à la partie de réception côté proximal (47).

15. Unité de traitement de préhension (20) selon la revendication 1, **caractérisée par le fait que** la partie de réception côté proximal (47) fait saillie vers un côté direction de fermeture (Y3) de la mâchoire (31) sur la surface de préhension (35).

16. Unité de traitement de préhension (20) selon la revendication 1, **caractérisée par le fait que** la contre-partie côté distal (46) forme une extrémité distale de la mâchoire (31) et s'étend sur une dimension de 0,5 mm ou plus et de 2,5 mm ou moins à partir de l'extrémité distale de la mâchoire (31) dans une direction longitudinale (C1, C2).
